# EUROPEAN PATENT APPLICATION

(11) **EP 4 744 583 A1**
(43) Date of publication of application: **20.05.2026**
(21) Application number: 25767258.4
(22) Date of filing: 27.02.2025
(51) Int. Cl.: A61B 5/021, A61B 5/0205

(54) **METHOD FOR MEASURING BLOOD PRESSURE, USER INTERFACE, AND RELATED DEVICE**

(30) Priority: 04.03.2024 CN 202410258478
(71) Applicant: Huawei Technologies Co., Ltd., Shenzhen, Guangdong 518129 (CN)
(72) Inventor: RONG, Meng, Shenzhen, Guangdong 518129 (CN); GAO, Yuxiang, Shenzhen, Guangdong 518129 (CN); LI, Hongbao, Shenzhen, Guangdong 518129 (CN); ZHU, Yu, Shenzhen, Guangdong 518129 (CN); ZHOU, Jie, Shenzhen, Guangdong 518129 (CN)
(74) Representative: Pfenning, Meinig & Partner mbB
(86) International application number: PCT/CN2025/079650
(87) International publication number: WO 2025/185519

(57) **Abstract**

This application discloses a blood pressure measurement method, a user interface, and a related apparatus. According to the method, after a blood pressure measurement operation is detected, whether a user has an irregular heartbeat symptom is determined based on historically collected first data and/or second data collected after the blood pressure measurement operation is detected, and when it is determined that the user has the irregular heartbeat symptom, a blood pressure value of the user is determined based on blood pressure values of a plurality of measurements. In this way, the user does not need to actively notify a device that the user has the irregular heartbeat symptom, to reduce inconvenience of a user operation, implement automatic recognition of a physical condition of the user by the device, measure a blood pressure in a targeted measurement manner based on the physical condition of the user, and implement a precise measurement of the blood pressure.

## Description

This application claims priority to Chinese Patent Application No. 202410258478.8, filed with the China National Intellectual Property Administration on March 4, 2024 and entitled "BLOOD PRESSURE MEASUREMENT METHOD, USER INTERFACE, AND RELATED APPARATUS", which is incorporated herein by reference in its entirety.

### TECHNICAL FIELD

This application relates to the field of terminal and computer technologies, and in particular, to a blood pressure measurement method, a user interface, and a related apparatus.

### BACKGROUND

There are many devices for measuring blood pressures in the market. Users can learn about their blood pressure statuses through these devices. However, for some users having an irregular heartbeat, changes of the irregular heartbeat make the blood pressures fluctuate more fiercely, and measurement results of the devices has large random errors, and cannot be used to reflect real blood pressure statuses of the users.

### SUMMARY

This application provides a blood pressure measurement method, a user interface, and a related apparatus, so that a device can automatically identify a physical condition of a user, and measure a blood pressure in a targeted measurement manner based on the physical condition of the user, without a need to actively report, by the user, that the user has an irregular heartbeat symptom.

According to a first aspect, an embodiment of this application provides a blood pressure measurement method, where the method is applied to an electronic device, and the method includes: detecting a blood pressure measurement operation; determining, based on historically collected first data and/or second data collected after the blood pressure measurement operation is detected, whether a user has an irregular heartbeat symptom; and when the user has the irregular heartbeat symptom, determining a blood pressure value of the user based on blood pressure values of a plurality of measurements.

According to the method provided in the first aspect, whether the user has the irregular heartbeat symptom can be evaluated based on the historical data of the user and/or the real-time data of the user, and the user does not need to actively notify the device of a physical condition of the user. This reduces inconvenience of a user operation, and implements automatic recognition of the physical condition of the user by the device. When the user has the irregular heartbeat, the blood pressure values of the plurality of measurements are used to accurately evaluate a blood pressure status of the user.

With reference to the first aspect, in an implementation, the second data is a photoplethysmography PPG signal or an oscillation wave signal.

In other words, the PPG signal or the oscillation wave signal collected during a blood pressure measurement may be used to accurately identify a heartbeat status of the user.

With reference to the first aspect, in an implementation, whether the user has the irregular heartbeat symptom is determined based on the second data collected after the blood pressure measurement operation is detected, and after detecting the blood pressure measurement operation, and before determining, based on the second data collected after the blood pressure measurement operation is detected, whether the user has the irregular heartbeat symptom, the method further includes: starting a 1^{st} blood pressure measurement, where the second data is data collected during the 1^{st} blood pressure measurement; and after determining, based on the second data collected after the blood pressure measurement operation is detected, whether the user has the irregular heartbeat symptom, the method further includes: when it is determined, based on the second data, that the user has the irregular heartbeat symptom, starting N blood pressure measurements after the 1^{st} blood pressure measurement ends, where the blood pressure values of the plurality of measurements include blood pressure values respectively obtained through the 1^{st} blood pressure measurement and the N blood pressure measurements, and N is a positive integer greater than or equal to 1; or when it is determined, based on the second data, that the user has the irregular heartbeat symptom, starting M blood pressure measurements after the 1^{st} blood pressure measurement ends, where the blood pressure values of the plurality of measurements include blood pressure values respectively obtained through the M blood pressure measurements, and M is a positive integer greater than or equal to 2.

It can be learned that if the electronic device identifies, based on the data collected after the blood pressure measurement operation is detected, whether the user has the irregular heartbeat symptom, the electronic device may start the 1^{st} blood pressure measurement after the blood pressure measurement operation is detected, and identify, based on the data collected during the blood pressure measurement, whether the user has the irregular heartbeat symptom. If it is identified that the user has the irregular heartbeat symptom, a quantity of blood pressure measurements may be added in time, so that the electronic device can determine the blood pressure value of the user based on the blood pressure values of the plurality of blood pressure measurements. In this way, the heartbeat status of the user can be identified during the blood pressure measurement, so that the electronic device can measure the blood pressure of the user in time in a blood pressure measurement manner for the heartbeat status of the user.

With reference to the first aspect, in an implementation, when the 1^{st} blood pressure measurement is started, a blood pressure measurement mode is a first mode, and a blood pressure measurement mode of the N blood pressure measurements is a second mode; and the electronic device is a wearable device configured with an airbag, and a pressure applied when the airbag is inflated in the first mode is lower than a pressure applied when the airbag is inflated in the second mode; and/or a blood pressure measurement model used in the first mode is different from a blood pressure measurement model used in the second mode, and the blood pressure measurement model is used to determine a blood pressure value of the measurement.

In other words, for different heartbeat statuses of the user, the electronic device may measure the blood pressure of the user in different blood pressure measurement modes, to implement a targeted blood pressure measurement based on a physical condition of the user, so as to improve accuracy of the blood pressure measurement.

With reference to the first aspect, in an implementation, after starting 1^{st} blood pressure measurement, the method further includes: during the 1^{st} blood pressure measurement, determining that the user has the irregular heartbeat symptom, and switching the blood pressure measurement mode of the 1^{st} blood pressure measurement from the first mode to the second mode.

Specifically, if the electronic device is a wearable device configured with an airbag, and the electronic device switches the blood pressure measurement mode from the low-pressure mode to the high-pressure mode during the blood pressure measurement, the electronic device may increase, during the blood pressure measurement, a maximum pressure applied when the airbag is inflated.

The blood pressure measurement model used in the first mode is a model established for healthy people, that is, not a model established for people having the irregular heartbeat, and the blood pressure measurement model used in the second mode is a model established for people having the irregular heartbeat.

If the electronic device identifies, based on the data collected during the blood pressure measurement, whether the user has the irregular heartbeat symptom, and the user identifies, during the blood pressure measurement, that the user has the irregular heartbeat symptom, the user may adjust a blood pressure measurement mode of the blood pressure measurement in time, so that the electronic device can complete the blood pressure measurement in a blood pressure measurement mode that meets the physical condition of the user.

With reference to the first aspect, in an implementation, the first data includes a PPG signal.

In other words, the PPG signal may be used to identify the heartbeat status of the user.

With reference to the first aspect, in an implementation, whether the user has the irregular heartbeat symptom is determined based on the historical first data of the user, and before determining the blood pressure value of the user based on the blood pressure values of the plurality of measurements for the user, the method further includes: starting a plurality of blood pressure measurements, where the blood pressure values of the plurality of measurements are blood pressure values obtained through the plurality of blood pressure measurements.

It can be learned that, if the electronic device identifies, based on the historical data, whether the user has the irregular heartbeat symptom, after the blood pressure measurement operation is detected, the electronic device may first identify, based on the historical data, whether the user has the irregular heartbeat symptom, and then measure the blood pressure of the user in the blood pressure measurement manner that meets the heartbeat status of the user, to accelerate a blood pressure measurement process.

With reference to the first aspect, in an implementation, the method further includes: when it is determined, based on the first data, that the user does not have the irregular heartbeat symptom, starting one blood pressure measurement, and determining a blood pressure value obtained through the one blood pressure measurement as the blood pressure value of the user.

In other words, if the electronic device determines, based on the historical data, that the user does not have the irregular heartbeat symptom, the electronic device may start only the one blood pressure measurement, and determine the blood pressure value obtained through the one blood pressure measurement as the blood pressure value of the user.

With reference to the first aspect, in an implementation, after starting the one blood pressure measurement, and before determining the blood pressure value obtained through the one blood pressure measurement as the blood pressure value of the user, the method further includes: determining, based on the second data collected during the one blood pressure measurement, that the user does not have the irregular heartbeat symptom.

When the electronic device identifies, based on the historical data, whether the user has the irregular heartbeat symptom, even if the electronic device determines, based on the historical data, that the user does not have the irregular heartbeat symptom, during an actual measurement, the electronic device may still continue to identify, based on the data collected during the measurement, whether the user has the irregular heartbeat symptom, to improve recognition accuracy of a heartbeat status.

With reference to the first aspect, in an implementation, the method further includes: when it is determined, based on the first data, that the user does not have the irregular heartbeat symptom, starting one blood pressure measurement; and when it is determined, based on the second data collected during the one blood pressure measurement, that the user has the irregular heartbeat symptom, starting N blood pressure measurements after the one blood pressure measurement ends, where the blood pressure values of the plurality of measurements include blood pressure values respectively obtained through the one blood pressure measurement and the N blood pressure measurements, and N is a positive integer greater than or equal to 1; or when it is determined, based on the second data, that the user has the irregular heartbeat symptom, starting M blood pressure measurements after a 1^{st} blood pressure measurement ends, where the blood pressure values of the plurality of measurements include blood pressure values respectively obtained through the M blood pressure measurements, and M is a positive integer greater than or equal to 2.

When the electronic device identifies, based on the historical data, whether the user has the irregular heartbeat symptom, even if the electronic device determines, based on the historical data, that the user does not have the irregular heartbeat symptom, during an actual measurement, the electronic device may still continue to identify, based on the data collected during the measurement, whether the user has the irregular heartbeat symptom, and adjust the blood pressure measurement mode in time when it is determined that the user has the irregular heartbeat, to avoid missed determining.

With reference to the first aspect, in an implementation, a blood pressure measurement mode of the one blood pressure measurement is a first mode, and a blood pressure measurement mode of the plurality of blood pressure measurements is a second mode; and the electronic device is a wearable device configured with an airbag, and a pressure applied when the airbag is inflated in the first mode is lower than a pressure applied when the airbag is inflated in the second mode; and/or a blood pressure measurement model used in the first mode is different from a blood pressure measurement model used in the second mode, and the blood pressure measurement model is used to determine a blood pressure value of the measurement.

The blood pressure measurement model used in the first mode is a model established for healthy people, that is, not a model established for people having the irregular heartbeat, and the blood pressure measurement model used in the second mode is a model established for people having the irregular heartbeat.

If the electronic device uses the historical data to identify whether the user has the irregular heartbeat symptom, for different heartbeat statuses of the user, the electronic device may measure the blood pressure of the user in different blood pressure measurement modes, to implement a targeted blood pressure measurement based on the physical condition of the user, so as to improve accuracy of the blood pressure measurement.

With reference to the first aspect, in an implementation, a deflation speed of the airbag in the first mode is greater than a deflation speed of the airbag in the second mode.

In other words, if the electronic device is a wearable device configured with the airbag, the deflation speed of the airbag in the high-pressure mode may be greater than the deflation speed of the airbag in the low-pressure mode. In this way, this can prevent the airbag from deflating quickly under a high inflation pressure in the high-pressure mode, to ensure comfort of the user during the measurement.

With reference to the first aspect, in an implementation, the blood pressure values of the plurality of measurements are determined based on an oscillation wave signal collected by the electronic device in an airbag inflation process.

In other words, the electronic device may collect, in an airbag inflation process, the data used for the blood pressure measurement, instead of collecting, in an airbag deflation process, the data used for the blood pressure measurement. This can shorten a time of blood pressure measurement and accelerate a speed of the blood pressure measurement.

With reference to the first aspect, in an implementation, in the blood pressure values of the plurality of measurements, a difference between blood pressure values of any two measurements is less than a first threshold, and/or a heart rate value of the user during each measurement is less than a second threshold, and/or a heart rate difference of the user during any two measurements is less than a third threshold.

In other words, a difference between data used for calculation of the blood pressure value of the user is small, and the electronic device may discard a measurement result with a large error during the blood pressure measurement, and calculate the blood pressure value of the user by using only a measurement result with a small error.

With reference to the first aspect, in an implementation, when the user has the irregular heartbeat symptom, after determining the blood pressure value of the user based on the blood pressure values of the plurality of measurements, the method further includes: displaying the blood pressure value of the user and a measurement identifier, where the measurement identifier indicates that the blood pressure value of the user is determined when the user has the irregular heartbeat symptom.

After the blood pressure of the user is measured, the electronic device may display the blood pressure value of the user, but also display a measurement state of the blood pressure measurement, so that the user can learn a situation in which the blood pressure measurement is completed, and have a more comprehensive understanding of the measurement result.

With reference to the first aspect, in an implementation, determining the blood pressure value of the user based on the blood pressure values of the plurality of measurements specifically includes: determining an average of the blood pressure values of the plurality of measurements as the blood pressure value of the user.

The measured blood pressure value is in a random fluctuation state when the user has an irregular heartbeat. In this case, the average of the blood pressure values of the plurality of measurements is determined as the blood pressure value of the user, so that problems of inaccurate measurement results caused by randomness of a blood pressure fluctuation can be reduced as much as possible, to accurately evaluate the blood pressure status of the user.

According to a second aspect, an embodiment of this application provides an electronic device, including a memory, one or more processors, and one or more programs. When the one or more processors execute the one or more programs, the electronic device is enabled to implement the method according to the second aspect or any implementation of the second aspect.

According to a third aspect, an embodiment of this application provides a computer-readable storage medium, including instructions. When the instructions are run on an electronic device, the electronic device is enabled to perform the method according to the first aspect or any possible implementation of the first aspect.

According to a fourth aspect, an embodiment of this application provides a computer program product. When the computer program product runs on a computer, the computer is enabled to perform the method according to the first aspect or any implementation of the first aspect.

### BRIEF DESCRIPTION OF DRAWINGS

FIG. 1A to FIG. 1J, FIG. 2, FIG. 3, FIG. 4, FIG. 5, FIG. 6, FIG. 7A, and FIG. 7B are some user interfaces according to embodiments of this application;
FIG. 8 is an overall schematic flowchart of a blood pressure measurement method according to an embodiment of this application;
FIG. 9 is a schematic flowchart of measuring, by an electronic device 100, a blood pressure in a non-guest mode according to an embodiment of this application;
FIG. 10 is a schematic flowchart of measuring, by an electronic device 100, a blood pressure in a guest mode according to an embodiment of this application;
FIG. 11 is a diagram of a hardware structure of an electronic device 100 according to an embodiment of this application; and
FIG. 12 is a diagram of a structure of a blood pressure measurement apparatus 200 according to an embodiment of this application.

### DESCRIPTION OF EMBODIMENTS

The following clearly and completely describes the technical solutions in embodiments of this application with reference to the accompanying drawings. In descriptions of embodiments of this application, unless otherwise specified, "/" indicates "or". For example, A/B may indicate A or B. The term "and/or" in this specification merely describes an association relationship between associated objects, and indicates that three relationships may exist. For example, A and/or B may indicate the following three cases: Only A exists, both A and B exist, and only B exists. In addition, in the descriptions of embodiments of this application, "a plurality of" means two or more.

The following terms "first" and "second" are merely intended for a purpose of description, and shall not be understood as an indication or implication of relative importance or implicit indication of a quantity of indicated technical features. Therefore, a feature limited by "first" or "second" may explicitly or implicitly include one or more features. In the descriptions of embodiments of this application, unless otherwise specified, "a plurality of" means two or more than two.

A term "user interface (user interface, UI)" in the following embodiments of this application is a medium interface for interaction and information exchange between an application or an operating system and a user, and implements conversion between an internal form of information and a form that can be accepted by the user. The user interface is source code written in a specific computer language like Java or an extensible markup language (extensible markup language, XML). Interface source code is parsed and rendered on an electronic device, and is finally presented as content that can be identified by the user. The user interface is usually represented in a form of graphical user interface (graphical user interface, GUI), and is a user interface that is related to a computer operation and that is displayed in a graphic manner. The user interface may be a visual interface element, for example, a text, an icon, a button, a menu, a tab, a text box, a dialog box, a status bar, a navigation bar, or a Widget that is displayed on a display of an electronic device.

An irregular heartbeat may be caused by the following plurality of cases.

### (1) Physiological condition

When a user is excited, drinks a large amount of alcohol or coffee, or exercises strenuously, the heart may be more excited, causing an irregular heartbeat symptom.

### (2) Pathological condition

Diseases of the user, such as atrial fibrillation, coronary heart disease, and myocarditis, may also cause the heart of the user to beat quickly and irregularly.

Atrial fibrillation, also known as fibrillation of a heart atrium, is a common arrhythmia in which a patient typically presents with rapid and irregular heart rate fluctuations. There is a blood pressure measurement device for the atrial fibrillation patient in the existing market. Before a measurement, the user may actively adjust a measurement mode to an atrial fibrillation mode, so that the blood pressure measurement device can measure a blood pressure value of the user in the atrial fibrillation mode, to implement a blood pressure measurement for the atrial fibrillation patient.

However, this method cannot implement imperceptible monitoring. The user needs to actively switch to the atrial fibrillation mode to measure a blood pressure of the user when the user knows that the user has an atrial fibrillation symptom. A user interaction process is not proper, and a blood pressure measurement process is complex.

An embodiment of this application provides a blood pressure measurement method. According to the method, after a blood pressure measurement operation is detected, whether a user has an irregular heartbeat symptom is determined based on historically collected first data and/or second data collected after the blood pressure measurement operation is detected, and when it is determined that the user has the irregular heartbeat symptom, a blood pressure value of the user is determined based on blood pressure values of a plurality of measurements.

It can be learned that, according to the blood pressure measurement method provided in this embodiment of this application, whether the user may have the irregular heartbeat symptom can be evaluated based on historical data of the user and/or real-time data of the user, and the user does not need to actively notify the device of the irregular heartbeat symptom of the user. This reduces inconvenience of a user operation, and implements automatic recognition of a physical condition of the user by the device. When the user has the irregular heartbeat, the blood pressure values of the plurality of measurements are used to accurately evaluate a blood pressure status of the user.

It may be understood that the blood pressure measurement method provided in this embodiment of this application is applicable to the blood pressure measurement of the user having the irregular heartbeat. The irregular heartbeat may be a symptom shown in a physiological condition or a pathological condition of the user. An atrial fibrillation patient, a user with excessive drinking, or the like can use the blood pressure measurement method provided in this embodiment of this application to implement blood pressure monitoring.

**The blood pressure measurement method provided in this embodiment of this application may be applied to the following electronic devices.**

### (1) Wearable device like a watch or a band

Because the wearable device may be worn on the user, and has a capability of collecting various physiological signals, where the physiological signal may be an electrocardiogram signal, a photoplethysmography (photoplethysmography, PPG) signal, an electromyographic signal, a skin electric response signal, or the like. Various physiological indicators of a human body, such as a blood pressure, a heart rate, blood oxygen saturation, and a body temperature, are calculated based on these signals.

Therefore, the blood pressure measurement method provided in this embodiment of this application may be applied to the wearable device. The user may start a blood pressure measurement by using the wearable device, identify, based on historical or real-time data collected by the wearable device, whether the user has the irregular heartbeat symptom, when it is determined that the user has the irregular heartbeat symptom, perform a plurality of measurements on the blood pressure of the user by using the wearable device, and output, on the wearable device, a final blood pressure result determined based on the blood pressures of the plurality of measurements.

### (2) Non-wearable device like a mobile phone or a tablet

Although the non-wearable device like a mobile phone or a tablet is not equipped to measure various body data of the user, the non-wearable device may establish a communication connection to the wearable device like a watch or a band, to obtain body data collected by the wearable device, and output a measurement result of a physiological parameter of the user.

Therefore, the blood pressure measurement result provided in this embodiment of this application may be applied to the non-wearable device. The user may start the blood pressure measurement by using the device, communicate with a wearable device to which a communication connection is established, obtain historical or real-time data collected by the wearable device, identify, based on the data, whether the user has the irregular heartbeat symptom, when it is determined that the user has the irregular heartbeat symptom, perform a plurality of measurements on the blood pressure of the user by using the wearable device, and output, on the non-wearable device, a final blood pressure result determined based on blood pressures of the plurality of measurements.

It may be understood that the blood pressure measurement method provided in this embodiment of this application may be further applied to another electronic device. Details are not described herein again.

**The following describes a principle of measuring a blood pressure by the electronic device 100.**

For example, the electronic device 100 may measure a blood pressure of the user in any one of the following manners.
(1) The electronic device 100 measures the blood pressure based on a collected PPG signal.

The PPG signal may be collected by using a photoelectric sensor on the wearable device.

The PPG signal represents a change in light absorption of skin tissue caused by blood flow. Specifically, when the heart contracts, a volume of blood in capillaries increases, light absorption is enhanced, and reflected light is weakened. On the contrary, when the heart relaxes, the volume of blood decreases, and emitted light is enhanced. Therefore, light is emitted to skin of the user by using the photoelectric sensor, and a change of the reflected light is analyzed, so that a vascular flow may be calculated, and a blood pressure value may be further obtained through analysis.

For example, the electronic device 100 is the wearable device. After starting the blood pressure measurement, the electronic device 100 may collect a PPG signal by using the photoelectric sensor, and obtain, through analysis based on the PPG signal, a blood pressure value obtained through this measurement.

For example, the electronic device 100 is the non-wearable device. After starting the blood pressure measurement, the electronic device 100 may obtain a PPG signal collected by the wearable device worn by the user, and obtain, through analysis based on the PPG signal, a blood pressure value obtained through this measurement.

In addition, the electronic device 100 may further analyze, based on the PPG signal, whether a heartbeat of the user is regular.

(2) The electronic device 100 measures the blood pressure based on a collected oscillation wave signal.

The oscillation wave signal may be collected by using a pressure sensor on a wearable device configured with an airbag.

The oscillation wave signal represents a pressure pulse transferred by arterial blood flow of the user under a pressure applied by the airbag. Specifically, the wearable device may compress the arterial blood flow by using the pressure applied by the airbag, then collect, by using the pressure sensor, an oscillation wave signal reflected by the arterial blood flow in a pressing case, and obtain, through analysis, the blood pressure based on a relationship between a fluctuation amplitude of the oscillation wave signal and the airbag pressure.

For example, the wearable device may be a device worn on a wrist of the user, for example, a watch or a band, and the airbag may be configured on a strap of the wearable device.

For example, the electronic device 100 is the wearable device. After starting the blood pressure measurement, the electronic device 100 may apply a pressure to the airbag, and then obtain, through analysis based on an oscillation wave signal collected by the pressure sensor in an airbag pressurization process, a blood pressure value obtained through this measurement. Alternatively, the electronic device 100 may obtain, through analysis based on an oscillation wave signal collected by the pressure sensor in an airbag deflation process, a blood pressure value obtained through this measurement.

For example, the electronic device 100 is the non-wearable device. After starting the blood pressure measurement, the electronic device 100 may obtain an oscillation wave signal collected by the wearable device worn by the user, and obtain, through analysis based on the oscillation wave signal, a blood pressure value obtained through this measurement.

In addition, the electronic device 100 may further analyze, based on the oscillation wave signal, whether a heartbeat of the user is regular.

It may be understood that the electronic device 100 may alternatively measure the blood pressure in another manner. This is not limited in embodiments of this application.

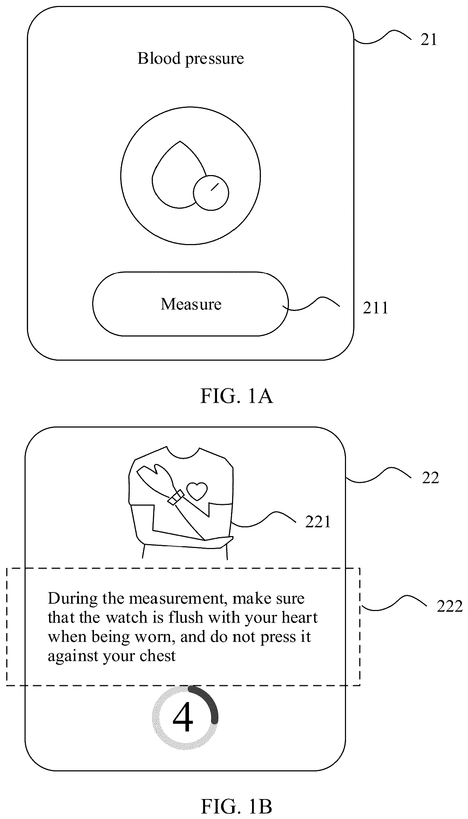

The following uses an example in which the blood pressure measurement method is applied to a wearable device, and the wearable device measures a blood pressure based on an oscillation wave signal, to describe some user interfaces in embodiments of this application with reference to FIG. 1A to FIG. 1J, FIG. 2, FIG. 3, FIG. 4, FIG. 5, FIG. 6, FIG. 7A, and FIG. 7B.

FIG. 1A to FIG. 1J show an example of related user interfaces used when the electronic device 100 identifies an irregular heartbeat symptom of a user based on data collected after a blood pressure measure operation is detected.

FIG. 1A is a user interface 21 displayed by the electronic device 100 according to an embodiment of this application. The user interface 21 may include a blood pressure measurement option 211. The blood pressure measurement option 211 may be used to trigger a measurement of a blood pressure of a user.

When the electronic device 100 detects a user operation performed on the blood pressure measurement option 211, for example, a tap operation, in response to the operation, the electronic device 100 may display a user interface 22 shown in FIG. 1B. The user interface 22 may be used to display a correct posture during the measurement to the user.

As shown in FIG. 1B, the user interface 22 may include a picture 221 and prompt information 222. The picture 221 may indicate the correct posture during the measurement to the user. The prompt information 222 may include a text: "During the measurement, make sure that the watch is flush with your heart when being worn, and do not press it against your chest".

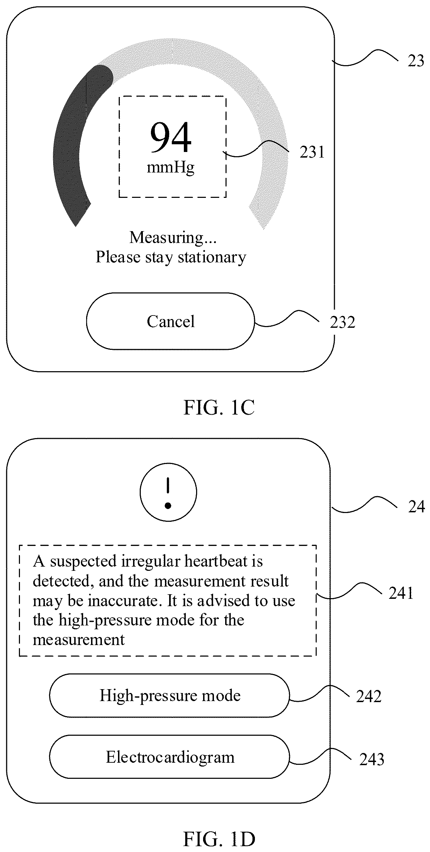

For example, after the user interface 22 is displayed for a period of time, for example, 4s, the electronic device 100 may start to measure the blood pressure of the user and display a user interface 23 shown in FIG. 1C.

As shown in FIG. 1C, the user interface 23 may include a pressure value 231 and a measurement cancellation option 232.

The pressure value 231 may be used to display an inflation pressure value of an airbag of the electronic device 100 during the measurement. For example, as shown in FIG. 1C, at a time point during the measurement, the pressure value 231 may be 94 mmHg. The measurement cancellation option 232 is used to trigger interruption of the blood pressure measurement. For example, if the electronic device 100 detects a user operation performed on the measurement cancellation option 232, for example, a tap operation, in response to the operation, the electronic device 100 may interrupt the measurement on the blood pressure of the user, and display the user interface 21 shown in FIG. 1A.

It may be understood that, after the electronic device 100 detects the user operation shown in FIG. 1A, the electronic device 100 may alternatively start the blood pressure measurement directly, that is, not display the user interface 22 shown in FIG. 1B, but directly display the user interface 23 shown in FIG. 1C. Further, when measuring the blood pressure of the user for a 1^{st} time, the electronic device 100 may display the user interface 22 shown in FIG. 1B, and when measuring the blood pressure of the user again subsequently, the electronic device 100 no longer display the user interface 22 shown in FIG. 1B.

During the blood pressure measurement of the electronic device 100, the electronic device 100 may determine, based on data collected by the electronic device 100, whether the user has an irregular heartbeat. If the electronic device 100 identifies that the user has the irregular heartbeat, a user interface 24 shown in FIG. 1D may be displayed, to prompt the user to switch to a high-pressure mode to measure the blood pressure.

The high-pressure mode is a blood pressure measurement mode for the user having the irregular heartbeat. In the high-pressure mode, the electronic device 100 may perform a plurality of measurements on the blood pressure of the user, and determine a blood pressure value of the user based on blood pressures of the plurality of measurements.

Correspondingly, before switching to the high-pressure mode, when starting the blood pressure measurement, a blood pressure measurement mode used by the electronic device 100 by default is a low-pressure mode.

The low-pressure mode is a blood pressure measurement mode for the user having a regular heartbeat. In the low-pressure mode, the electronic device 100 only needs to perform one measurement on the blood pressure of the user, and determine the blood pressure value of the one measurement as the blood pressure value of the user.

In some implementations, if the electronic device 100 is a wearable device configured with an airbag, during the blood pressure measurement, in comparison with the low-pressure mode, in the high-pressure mode, a time for inflating the airbag is longer, and a pressure for pressurizing the airbag is higher. Due to a longer inflation time and a higher pressurization pressure of the airbag, the electronic device 100 can collect more oscillation wave signals, and calculate the blood pressure value based on the more oscillation wave signals, so that more detailed information can be extracted, and a blood pressure value obtained through the measurement is more accurate. In addition, it is considered that the low-pressure mode is a blood pressure measurement mode used by normal people having a regular heartbeat, the blood pressure of the user usually fluctuates regularly, so that the blood pressure value of the user can also be accurately measured although a small quantity of oscillation wave signals are collected. Therefore, if the electronic device 100 detects that the user has the irregular heartbeat symptom, an inflation pressure of the airbag may be increased, so that the electronic device 100 can collect more accurate data for analyzing the blood pressure value of the user.

Further, a deflation speed of the airbag in the high-pressure mode may be less than a deflation speed of the airbag in the low-pressure mode. In this way, this can prevent the airbag from deflating excessively quickly under an excessively high pressure in the high-pressure mode, to reduce discomfort of the user during the blood pressure measurement.

As shown in FIG. 1D, the user interface 24 may include prompt information 241, an option 242, and an option 243.

The prompt information 241 may be used to inform the user that it is advised to switch to the high-pressure mode to measure the blood pressure. For example, the prompt information 241 may display a text "A suspected irregular heartbeat is detected, and the measurement process may be inaccurate. It is advised to use the high-pressure mode for the measurement".

The option 242 may be used to trigger entering the high-pressure mode to measure the blood pressure. If the electronic device 100 detects a user operation performed on the option 242, for example, a tap operation, in response to the operation, the electronic device 100 may switch currently performed the blood pressure measurement from the low-pressure mode to the high-pressure mode. Specifically, the electronic device 100 may change a process in which only one blood pressure measurement needs to be performed to a process in which three blood pressure measurements need to be performed. Further, when detecting that the user has the irregular heartbeat symptom, the electronic device 100 may increase the inflation pressure of the airbag during the blood pressure measurement, and reduce the deflation speed of the airbag.

The option 243 may be used to trigger an electrocardiogram measurement. If the electronic device 100 detects a user operation performed on the option 243, for example, a tap operation, in response to the operation, the electronic device 100 may start the electrocardiogram measurement, to measure an electrocardiogram of the user, and output an evaluation result of the electrocardiogram of the user. The user may determine, based on the evaluation result, whether the user actually has the irregular heartbeat symptom. In this way, when the electronic device 100 identifies that the heartbeat of the user is irregular, whether the user has the irregular heartbeat symptom can be further verified based on the evaluation result of the electrocardiogram, to avoid a misdiagnosis.

The user interface 24 shown in FIG. 1D may have the following two display occasions.
(1) The user interface 24 shown in FIG. 1D may be displayed during the blood pressure measurement.

To be specific, if the electronic device 100 detects, during the blood pressure measurement, that the user has the irregular heartbeat symptom, the electronic device 100 may display the user interface 24 shown in FIG. 1D before this blood pressure measurement ends.

In this case, the electronic device 100 may directly switch the currently performed blood pressure measurement from the low-pressure mode to the high-pressure mode, that is, increase the inflation pressure of the airbag, reduce the deflation speed of the airbag during the current measurement, and continue to perform two blood pressure measurements in the high-pressure mode after the measurement ends. In this way, the electronic device 100 may calculate the blood pressure value of the user based on blood pressure values obtained through three measurements in the high-pressure mode.

(2) The user interface 24 shown in FIG. 1D may alternatively be displayed after the blood pressure measurement ends.

To be specific, if the electronic device 100 has completed one blood pressure measurement when detecting that the user has the irregular heartbeat symptom, the electronic device 100 may display the user interface 24 shown in FIG. 1D after the blood pressure measurement ends.

In this case, the electronic device 100 may perform two blood pressure measurements in the high-pressure mode after a 1^{st} blood pressure measurement ends. In this way, the electronic device 100 may calculate the blood pressure value of the user based on the blood pressure value obtained through one measurement in the low-pressure mode and the blood pressure values of two measurements in the high-pressure mode; or the electronic device 100 may perform three blood pressure measurements in the high-pressure mode after the 1^{st} blood pressure measurement ends. In this way, the electronic device 100 may calculate the blood pressure value of the user based on the blood pressure values of three measurements in the high-pressure mode.

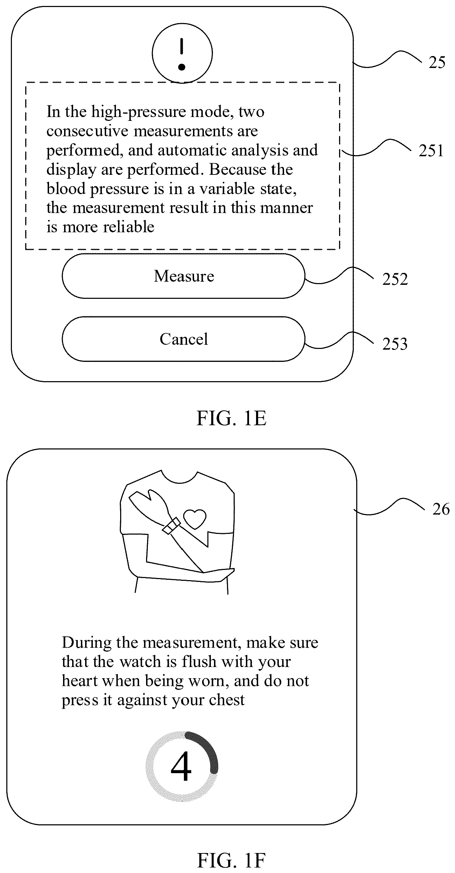

For example, if the electronic device 100 detects the user operation performed on the option 242, the electronic device 100 may display a user interface 25 shown in FIG. 1E.

As shown in FIG. 1E, the user interface 25 may include prompt information 251, a measurement start option 252, and a measurement cancellation option 253.

The prompt information 251 may be used to prompt the user with precautions in the high-pressure mode. For example, the prompt information 251 may include a text "In the high-pressure mode, two consecutive measurements are performed, and automatic analysis and display are performed. Because the blood pressure is in a variable state, the measurement result in this manner is more reliable".

The measurement start option 252 may be used to trigger start of the blood pressure measurement in the high-pressure mode. For example, if the electronic device 100 detects a user operation performed on the measurement start option 252, for example, a tap operation, the electronic device 100 may measure the blood pressure of the user in the high-pressure mode.

The measurement cancellation option 253 may be used to trigger measurement cancellation. For example, if the electronic device 100 detects a user operation performed on the measurement cancellation option 253, the electronic device 100 may keep performing a blood pressure measurement in the low-pressure mode, and after the blood pressure measurement ends, use a blood pressure value obtained through this measurement as the blood pressure value of the user.

When the electronic device 100 detects a user operation performed on the measurement start option 252, in response to the operation, the electronic device 100 determines to start the blood pressure measurement in the high-pressure mode.

If the electronic device 100 has completed one blood pressure measurement, the electronic device 100 may start another blood pressure measurement, and a blood pressure measurement mode of the started another blood pressure measurement is the high-pressure mode. For example, the electronic device 100 may display a user interface 26 shown in FIG. 1F. The user interface 26 may be used to display the correct posture during the measurement to the user.

For specific descriptions of the user interface 26, refer to related content of the user interface 22 in FIG. 1B. Details are not described herein again.

It may be understood that, in a process in which the electronic device 100 measures the blood pressure, if the electronic device 100 detects that the user has the irregular heartbeat symptom, the electronic device 100 may directly automatically switch to the high-pressure mode to measure the blood pressure without a need to display the user interfaces shown in FIG. 1D and FIG. 1E, to reduce inconvenience of a user operation.

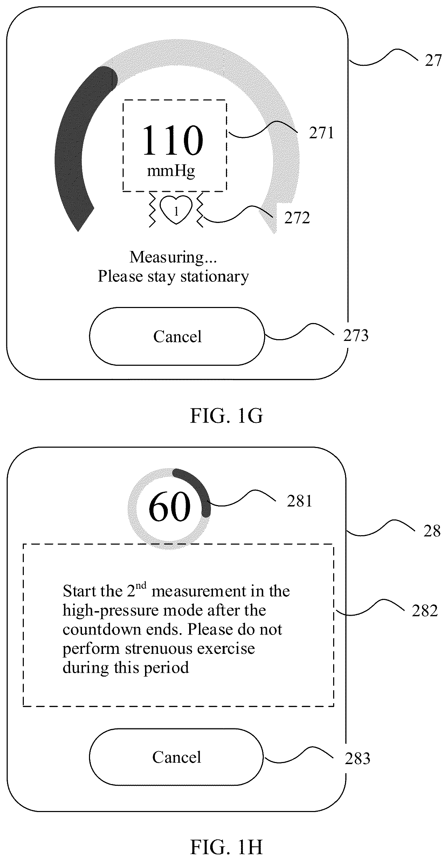

After displaying the user interface 26 shown in FIG. 1F for a period of time, for example, 4s, the electronic device 100 starts to measure the blood pressure of the user, and displays a user interface 27 shown in FIG. 1G.

As shown in FIG. 1G, the user interface 27 may include a pressure value 271, a mode identifier 272, and a measurement cancellation option 273.

The pressure value 271 may be used to display the inflation pressure value of the airbag of the electronic device 100 during the measurement. For example, as shown in FIG. 1G, at a time point during the measurement, the pressure value 271 may be 110 mmHg. The mode identifier 272 may indicate the high-pressure mode, and the user may learn, based on the mode identifier 272, that the user is currently measuring the blood pressure in the high-pressure mode. The measurement cancellation option 273 may be used to trigger interruption of the blood pressure measurement. For example, if the electronic device 100 detects a user operation performed on the measurement cancellation option 273, for example, a tap operation, in response to the operation, the electronic device 100 may interrupt the measurement on the blood pressure, and display the user interface 21 shown in FIG. 1A.

Then, after this blood pressure measurement in the high-pressure mode ends, the electronic device 100 may display a user interface 28 shown in FIG. 1H.

As shown in FIG. 1H, the user interface 28 may include a countdown 281, prompt information 282, and a measurement cancellation option 283.

The countdown 281 may be used to display a countdown of preset duration. For example, the preset duration may be 60s. The electronic device 100 may start the blood pressure measurement in the high-pressure mode after the countdown ends.

The prompt information 282 may be used to inform the user that one more blood pressure measurement needs to be performed in the high-pressure mode. For example, the prompt information 282 may include a text "Start the 2^{nd} measurement in the high-pressure mode after the countdown ends. Please do not perform strenuous exercise during this period".

The measurement cancellation option 283 may be used to trigger measurement cancellation. For example, if the electronic device 100 detects a user operation performed on the measurement cancellation option 283, the electronic device 100 may output a blood pressure result obtained through a latest blood pressure measurement, or the electronic device 100 may summarize a plurality of blood pressure results measured in response to the user operation performed on the blood pressure measurement option 211 shown in FIG. 1A, and output a summarized blood pressure result.

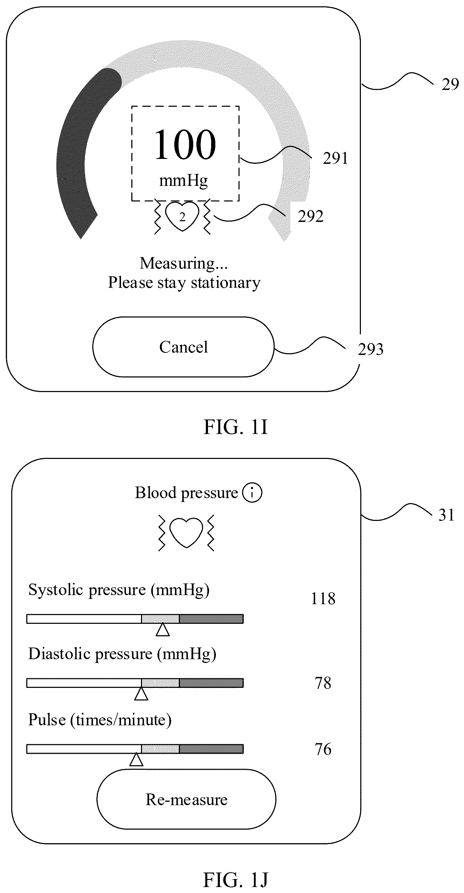

After the countdown in the countdown 281 shown in FIG. 1H is reset to zero, the electronic device 100 may start a 3^{rd} blood pressure measurement, and display a user interface 29 shown in FIG. 1I.

As shown in FIG. 1I, the user interface 29 may include a pressure value 291, a mode identifier 292, and a measurement cancellation option 293.

The pressure value 291 may be used to display the inflation pressure value of the airbag of the electronic device 100 during the measurement. For example, as shown in FIG. 1I, at a time point during the measurement, the pressure value 291 may be 100 mmHg. The mode identifier 292 may indicate the high-pressure mode. The measurement cancellation option 293 may be used to trigger interruption of the blood pressure measurement. For specific descriptions of the user interface 29, refer to related content of the user interface 27 described in FIG. 1G. Details are not described herein again.

After the electronic device 100 completes three measurements, the electronic device 100 may summarize blood pressure results of the plurality of measurements, and display a user interface 31 shown in FIG. 1J. The user interface 31 may be used to display the blood pressure result summarized by the electronic device 100. The measured blood pressure result summarized by the electronic device 100 may include a plurality of values such as a systolic pressure, a diastolic pressure, and a pulse.

As shown in FIG. 1J, the user interface 31 may be used to display values of the systolic pressure, the diastolic pressure, and the pulse. For example, the value of the systolic pressure may be an average of systolic pressures of the plurality of measurements, the value of the diastolic pressure may be an average of diastolic pressures of the plurality of measurements, and the value of the pulse may be an average of pulses of the plurality of measurements. As shown in FIG. 1J, the user interface 31 may show that the value of the systolic pressure of the user is 118 mmHg, the value of the diastolic pressure of the user is 78 mmHg, and the value of the pulse of the user is 76 times/minute.

The electronic device 100 may input, into a blood pressure measurement model, data collected during a blood pressure measurement, for example, an oscillation wave signal, to obtain a blood pressure value of the blood pressure measurement.

In some implementations, a blood pressure measurement model used in the low-pressure mode may be different from a blood pressure measurement model used in the high-pressure mode. The blood pressure measurement model used in the low-pressure mode is not a model established for people having an irregular heartbeat, and the model may be obtained by performing training on blood pressure data of people having a regular heartbeat. The blood pressure measurement model used in the high-pressure mode is a model established for the people having the irregular heartbeat, and the model may be obtained by performing training on blood pressure data of the people having the irregular heartbeat. In this way, in different blood pressure measurement modes, the electronic device 100 may calculate the blood pressure value in different blood pressure measurement modes, to improve accuracy of the blood pressure measurement.

In some implementations, after obtaining a result of each blood pressure measurement, the electronic device 100 may display a user interface similar to the user interface 31 shown in FIG. 1J, to display the blood pressure result obtained by the user during the blood pressure measurement.

It may be understood that, in FIG. 1A to FIG. 1J, three blood pressure measurements are used as an example, to describe a process in which the electronic device 100 measures the blood pressure when recognizing that the user has the irregular heartbeat symptom. In another embodiment of this application, the electronic device 100 may further perform more or fewer blood pressure measurements in the high-pressure mode. A quantity of blood pressure measurements in the high-pressure mode is not limited in embodiments of this application.

In addition, if the blood pressure result of the 1^{st} blood pressure measurement is a blood pressure result obtained in the low-pressure mode, the electronic device 100 may not use the blood pressure result obtained in the low-pressure mode to calculate the final blood pressure result, but only use the blood pressure result obtained in the high-pressure mode to perform calculation. In addition, if the three blood pressure measurements are used as an example, the electronic device 100 may use the blood pressure results obtained through the three measurements in the high-pressure mode to calculate the final blood pressure result.

It can be learned from FIG. 1A to FIG. 1J that, after the electronic device 100 detects the user operation of starting blood pressure measurement by the user, the electronic device 100 may start the blood pressure measurement in the low-pressure mode by default, detect, during the measurement, whether the user has the irregular heartbeat symptom, if the user has the irregular heartbeat symptom, switch the low-pressure mode to the high-pressure mode, measure the blood pressure of the user in the high-pressure mode, which may be specifically reflected in increasing a quantity of blood pressure measurements, and after the measurements end, output a blood pressure result obtained by perform calculation on blood pressure results of the plurality of measurements, so that the user can obtain an accurate blood pressure result when the user has the irregular heartbeat symptom.

In addition, after starting the blood pressure measurement, the electronic device 100 detects, based on data collected in real time, whether the user has the irregular heartbeat symptom. Further, after detecting the operation of starting the blood pressure measurement by the user, the electronic device 100 may further determine, based on historical data of the user, whether the user has ever had the irregular heartbeat symptom, so that when the blood pressure measurement is started, a corresponding blood pressure measurement mode is directly used to measure the blood pressure of the user.

The following provides descriptions by using examples with reference to FIG. 1A to FIG. 1J.

After the electronic device 100 detects the user operation performed on the blood pressure measurement option 211 shown in FIG. 1A, the electronic device 100 may obtain data related to the blood pressure of the user within preset duration (for example, 30 minutes) before a current time point, for example, a PPG signal, and the electronic device 100 may determine, based on the data, whether the user has ever had the irregular heartbeat symptom. If the user has ever had the irregular heartbeat symptom, the blood pressure measurement in the high-pressure mode may be started. If the user has never had the irregular heartbeat symptom, the blood pressure measurement in the low-pressure mode may be started.

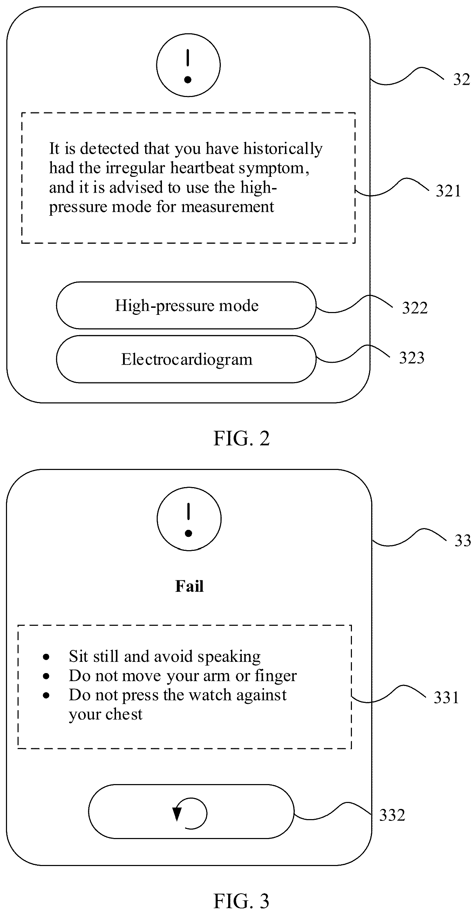

For example, if the electronic device 100 identifies that the user was in an irregular heartbeat state, the electronic device 100 may display a user interface 32 shown in FIG. 2.

As shown in FIG. 2, the user interface 32 may include prompt information 321, an option 322, and an option 323.

The prompt information 321 may be used to inform the user that it is advised to use the high-pressure mode to measure the blood pressure. For example, the prompt information 321 may display a text "It is detected that you have historically had the irregular heartbeat symptom, and it is advised to use the high-pressure mode for the measurement".

The option 322 may be used to trigger entering the high-pressure mode to measure the blood pressure. If the electronic device 100 detects a user operation performed on the option 322, for example, a tap operation, in response to the operation, the electronic device 100 may start the blood pressure measurement in the high-pressure mode, which may specifically include performing three measurements on the blood pressure of the user in the high-pressure mode. Further, if the electronic device 100 is the wearable device configured with the airbag, during the measurement, the electronic device 100 may apply a high inflation pressure to the airbag, and deflate the airbag at a slow speed. For a specific blood pressure measurement process in the high-pressure mode, refer to related descriptions of FIG. 1A to FIG. 1I. Details are not described herein again.

The option 323 may be used to trigger an electrocardiogram measurement. For specific descriptions of the option 323, refer to related content of the option 243 in FIG. 1D. Details are not described herein again.

For example, if the electronic device 100 detects a user operation performed on the option 322, in response to the operation, the electronic device 100 may start the blood pressure measurement in the high-pressure mode, display the user interface 27 shown in FIG. 1F, and display the blood pressure value measured by the electronic device 100 in real time. Then, after the electronic device 100 completes three blood pressure measurements in the high-pressure mode, the electronic device 100 may determine a final blood pressure result of the user based on results of the three blood pressure measurements, and display a user interface similar to the user interface 31 shown in FIG. 1J.

In other words, after detecting the user operation of starting the blood pressure measurement by the user, the electronic device 100 may analyze, based on the historical data of the user, whether the user has historically had the irregular heartbeat, and directly measure the blood pressure of the user in the corresponding blood pressure measurement mode based on a physical condition of the user, without a need to detect whether the user has ever had the irregular heartbeat during the measurement, to reduce inconvenience of switching the blood pressure measurement mode during the blood pressure measurement of the electronic device 100.

In some implementations, the electronic device 100 may determine, by combining the historical data and data collected after the blood pressure measurement operation is detected, whether the user has the irregular heartbeat symptom, to avoid missed determining of the symptom. This is because even if no irregular heartbeat symptom of the user is detected based on the historical data, the user may still have the irregular heartbeat symptom during the blood pressure measurement, or even if no irregular heartbeat symptom of the user is detected during the blood pressure measurement, the user may have the irregular heartbeat symptom in a historical time period before the measurement of the user.

Therefore, after the electronic device 100 detects the user operation of starting the blood pressure measurement by the user, if it is not detected, based on the historical data of the user, that the user has historically had the irregular heartbeat, the electronic device 100 may first measure the blood pressure in the low-pressure mode, and during the blood pressure measurement, detect whether the user has the irregular heartbeat symptom. If the user has the irregular heartbeat symptom, the electronic device 100 may switch to the high-pressure mode to measure the blood pressure. Alternatively, after the electronic device 100 detects the user operation of starting the blood pressure measurement by the user, if the electronic device 100 does not detect that the user has the irregular heartbeat symptom during the blood pressure measurement in the low-pressure mode, but identifies, based on the historical data, that the user has historically had the irregular heartbeat symptom, the electronic device 100 may switch to the high-pressure mode to measure the blood pressure.

A blood pressure measurement process is easily affected by external factors, for example, a user measurement posture and a user mood, resulting in a measurement failure or a large measurement error. Therefore, during the measurement or after the measurement ends, the electronic device 100 may output prompt information, to inform the user that a measurement result may be inaccurate or prompt the user to perform a re-measurement.

FIG. 3 shows a user interface 33 displayed by the electronic device 100 after it is detected that the blood pressure measurement fails during the blood pressure measurement.

In a specific implementation, during the blood pressure measurement, the electronic device 100 may detect whether the user measurement posture changes, whether a difference between blood pressure data of the current measurement and blood pressure data of the previous measurement is excessively large, or the like. If it is detected that the user changes the measurement posture, for example, swings an arm, or detects that the difference between the blood pressure data of the measurement and the blood pressure data of the last measurement is excessively large, the electronic device 100 may display the user interface 33 shown in FIG. 3.

As shown in FIG. 3, the user interface 33 may include prompt information 331 and a re-measurement option 332.

The prompt information 331 may be used to prompt the user with precautions during the measurement. For example, the prompt information 331 may include: "Sit still and avoid speaking; do not move your arm or finger; and do not press the watch against your chest".

The re-measurement option 332 may be used to trigger a re-measurement of the blood pressure. If the electronic device 100 detects a user operation performed on the re-measurement option 332, the electronic device 100 does not need to use, for calculation of the finally determined blood pressure result, a blood pressure measurement result obtained after the current measurement fails, and restart the blood pressure measurement.

For example, in a process in which the electronic device 100 displays FIG. 1A to FIG. 1J, if the electronic device 100 detects that a 2^{nd} blood pressure measurement fails, the electronic device 100 may display the user interface 33 shown in FIG. 3 after displaying the user interface 27 shown in FIG. 1G, and when the electronic device 100 detects the user operation performed on the re-measurement option 332 shown in FIG. 3, for example, a tap operation, in response to the operation, the electronic device 100 may restart the 2^{nd} blood pressure measurement.

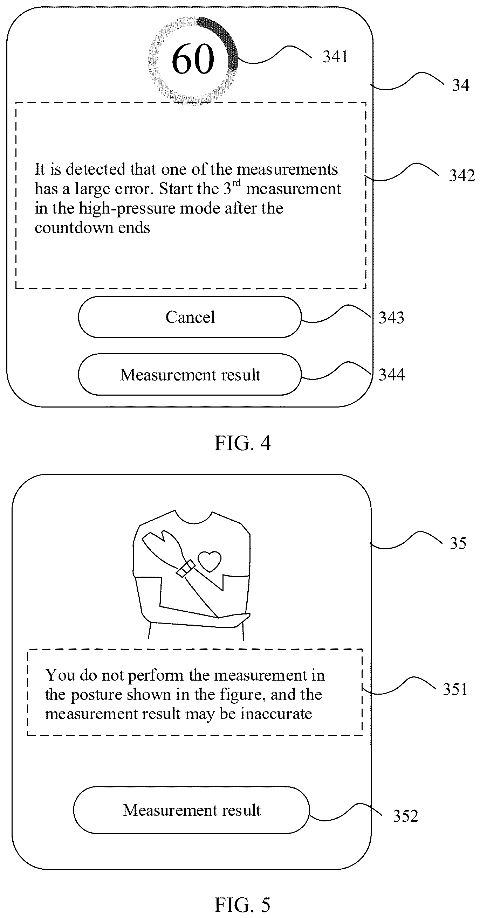

FIG. 4 shows a user interface 34 displayed when the electronic device 100 detects that an error of one of the plurality of blood pressure measurements is large after the plurality of blood pressure measurements in the high-pressure mode end.

In a specific implementation, because the electronic device 100 may perform the plurality of measurements on the blood pressure in the high-pressure mode, after the measurements end, the electronic device 100 may identify, based on the plurality of blood pressure results, whether there is a blood pressure result with a large error in the plurality of blood pressure results. If there is the blood pressure result, the electronic device 100 may display the user interface 34 shown in FIG. 4.

For example, during the plurality of blood pressure measurements, if a difference between a blood pressure value of one measurement in the measurements and a blood pressure value of any other measurement is less than a threshold (for example, 10), and/or if a heart rate value of the user during the one blood pressure measurement is less than a threshold (for example, 90), and/or a difference between the heart rate value of the user during the one measurement and a heart rate value of the user during any other measurement is less than a threshold (for example, 10), it indicates that the blood pressure value of the one measurement is normal, otherwise, it indicates that an error of the blood pressure value of the one measurement is large.

As shown in FIG. 4, the user interface 34 may include a countdown 341, prompt information 342, a measurement cancellation option 343, and a measurement result viewing option 344.

The countdown 341 may be used to display a countdown of a period of time (for example, 60s).

The prompt information 342 may be used to prompt the user to perform another blood pressure measurement in the high-pressure mode. For example, the prompt information 342 may include: "It is detected that one of the measurements has a large error. Start the 3^{rd} measurement in the high-pressure mode after the countdown ends".

The measurement cancellation option 343 may be used to trigger cancellation of the blood pressure measurement. For example, if the electronic device 100 detects a user operation performed on the measurement cancellation option 343, for example, a tap operation, in response to the operation, the electronic device 100 may display the user interface 21 shown in FIG. 1A.

The measurement result viewing option 344 may be used to trigger cancellation of the another blood pressure measurement, and trigger displaying, by the electronic device 100, a blood pressure result obtained through the measurement in the high-pressure mode.

If the electronic device 100 does not detect, within a period of time in which the user interface 34 is displayed, a user operation performed by the user on the measurement cancellation option 343 or the measurement result viewing option 344, the electronic device 100 may trigger another blood pressure measurement in the high-pressure mode after the countdown displayed in the countdown 341 is reset to zero.

For example, with reference to FIG. 1A to FIG. 1J, after completing the three measurements, that is, after displaying FIG. 1I and before displaying FIG. 1J, the electronic device 100 may display FIG. 4, to prompt the user to perform another blood pressure measurement in the high-pressure mode.

If the electronic device 100 detects, during the blood pressure measurement or after the blood pressure measurement ends, that the user does not measure the blood pressure in the correct posture, the electronic device 100 may display a user interface 35 shown in FIG. 5.

As shown in FIG. 5, the user interface 35 may include prompt information 351 and a measurement result viewing option 352.

The prompt information 351 may be used to inform the user that a measurement posture during the current blood pressure measurement is incorrect. For example, the prompt information 351 may include: "You do not perform the measurement in the posture shown in the figure, and the measurement result may be inaccurate".

The measurement result viewing option 352 may be used to trigger viewing of a result of the blood pressure measurement. For example, if the electronic device 100 detects a user operation performed on the measurement result viewing option 352, for example, a tap operation, in response to the operation, the electronic device 100 may display the user interface 31 shown in FIG. 1J.

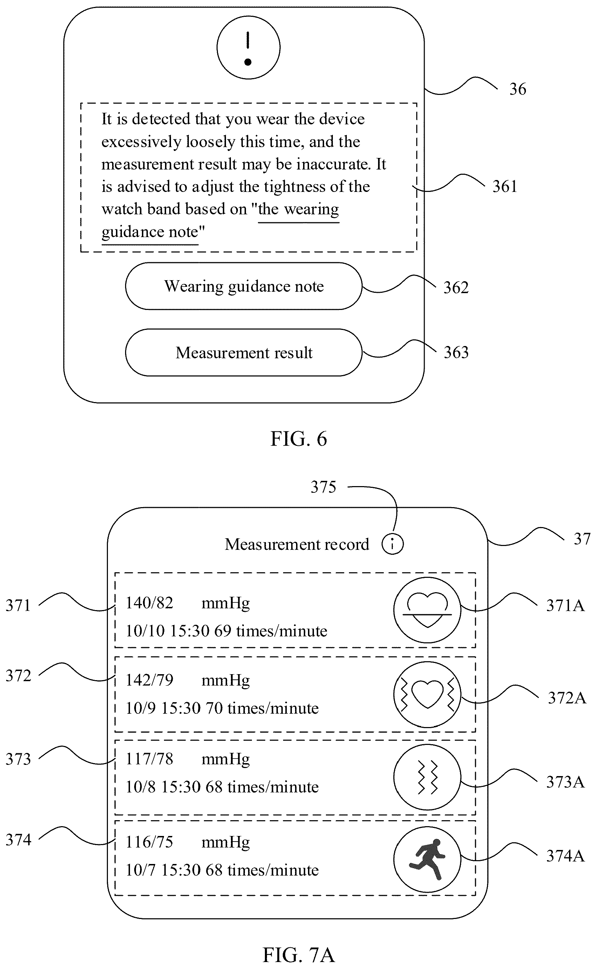

If the electronic device 100 detects that the device is worn excessively loosely during the blood pressure measurement, the electronic device 100 may display a user interface 36 shown in FIG. 6 during the blood pressure measurement or after the blood pressure measurement ends.

As shown in FIG. 6, the user interface 36 may include prompt information 361, a wearing guidance note option 362, and a measurement result viewing option 363.

The prompt information 361 may be used to inform the user that the device is worn excessively loosely during this blood pressure measurement. For example, the prompt information 361 may include: "It is detected that you wear the device excessively loosely this time, and the measurement result may be inaccurate. It is advised to adjust the tightness of the watch band based on "the wearing guidance note"".

The wearing guidance note option 362 may be used to display a guidance note of the worn device, and the user may correct a wearing manner of the device based on the guidance note.

The measurement result viewing option 363 may be used to trigger viewing of a result of the blood pressure measurement. For example, if the electronic device 100 detects a user operation performed on the measurement result viewing option 363, for example, a tap operation, in response to the operation, the electronic device 100 may display the user interface 31 shown in FIG. 1J.

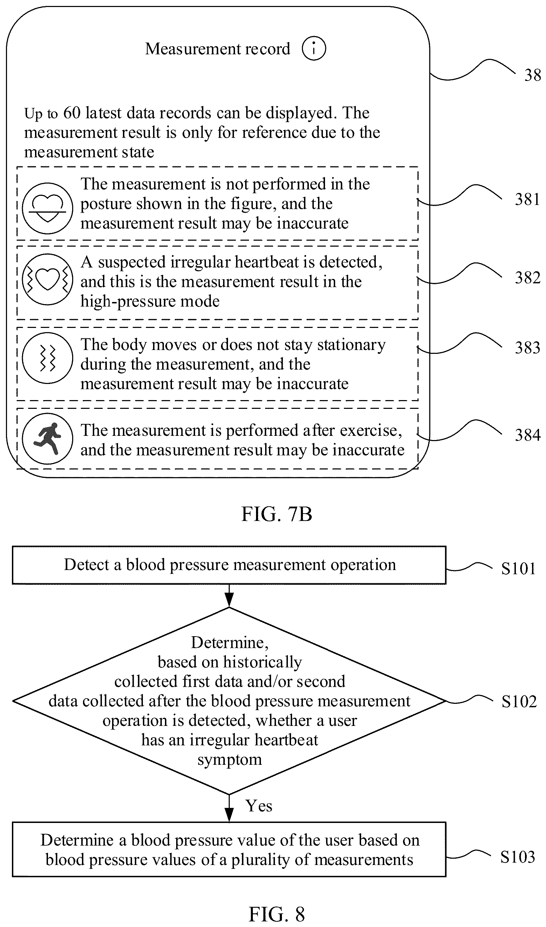

FIG. 7A and FIG. 7B show related user interfaces used when the electronic device 100 displays results of a plurality of blood pressure measurements.

FIG. 7A shows a user interface 37 used by the electronic device 100 to display the results of the plurality of blood pressure measurements. The user interface 37 may be used to display one or more blood pressure result options, and the blood pressure result option may be used to display related information of one blood pressure measurement, including but not limited to a blood pressure result, a measurement time, and the like.

As shown in FIG. 7A, the user interface 37 may include a blood pressure result option 371, a blood pressure result option 372, a blood pressure result option 373, and a blood pressure result option 374. The blood pressure result option 371 is used as an example. The blood pressure result option 371 is used to display a blood pressure result measured by the user at 15:30 on October 10. The blood pressure result may include: a value of a systolic pressure of 140 mmHg, a value of a diastolic pressure of 82 mmHg, and a pulse of 69 times/minute.

In addition, the blood pressure result option may further include a measurement identifier, and the measurement identifier indicates a measurement state of this blood pressure measurement.

As shown in FIG. 7A, the blood pressure result option 371 may include a measurement identifier 371A, and the measurement identifier 371A may indicate that the user does not measure the blood pressure in the correct posture during this measurement. The blood pressure result option 372 may include a measurement identifier 372A, and the measurement identifier 372A may indicate that the measurement is performed in the high-pressure mode. The blood pressure result option 373 may include a measurement identifier 373A, and the measurement identifier 373A may indicate that the body of the user moves or does not stay stationary during the measurement. The blood pressure result option 374 may include a measurement identifier 374A, and the measurement identifier 374A may indicate that the blood pressure measurement indicates a measurement result after the user exercises.

For example, the user interface 37 may further include an identifier 375, and the identifier 375 may be used to trigger viewing of meanings of all measurement identifiers in the user interface 37. If the electronic device 100 detects a user operation performed on the identifier 375, for example, a tap operation, the electronic device 100 may display a user interface 38 shown in FIG. 7B.

As shown in FIG. 7B, the user interface 38 may include an identifier description 381, an identifier description 382, an identifier description 383, and an identifier description 384. The identifier description 381 is used to display a meaning of the measurement identifier 371A shown in FIG. 7A. For example, the identifier description 381 may display "The measurement is not performed in the posture shown in the figure, and the measurement result may be inaccurate". The identifier description 382 is used to display a meaning of the measurement identifier 372A shown in FIG. 7A. For example, the identifier description 382 may display "A suspected irregular heartbeat is detected, and this is the measurement result in the high-pressure mode". The identifier description 383 is used to display a meaning of the measurement identifier 373A shown in FIG. 7A. For example, the identifier description 383 may display "The body moves or does not stay stationary during the measurement, and the measurement result may be inaccurate". The identifier description 384 is used to display a meaning of the measurement identifier 374A shown in FIG. 7A. For example, the identifier description 384 may display "The measurement is performed after exercise, and the measurement result may be inaccurate".

It may be understood that the measurement identifier included in the blood pressure result option may be identified by the electronic device 100 with reference to data of the user, and the data may include but is not limited to: a placement position of a user arm, blood pressure data measured in real time, historical blood pressure data of the user, a breathing frequency, a heartbeat frequency, a skin temperature, and the like.

Further, in addition to the measurement identifiers shown in FIG. 7A, the electronic device 100 may further identify another measurement state, and display a measurement identifier corresponding to the measurement state in the blood pressure result option shown in FIG. 7A. For example, the electronic device 100 may further identify whether the device is worn excessively loosely, and if a result of one of the measurements is obtained when the device is worn excessively loosely, a measurement identifier corresponding to being worn excessively loosely may be displayed in a result option corresponding to the result of the measurement.

In some implementations, considering that there may be a plurality of measurement states during a same blood pressure measurement, the electronic device 100 may set different priorities for different measurement states. In this way, when the electronic device 100 displays a measurement identifier corresponding to a result of one blood pressure measurement, the electronic device 100 may select a measurement state with a highest priority from the plurality of measurement states in the result of the blood pressure measurement. For example, a priority of the measurement identifier 373A shown in FIG. 7A may be higher than a priority of the measurement identifier 372A shown in FIG. 7A. In other words, during a same blood pressure measurement, if the electronic device 100 identifies that the user may have the irregular heartbeat, and the body moves or does not stay stationary during the measurement, the electronic device 100 preferentially determines that a result of the blood pressure measurement is obtained when the body moves or does not stay stationary during the measurement, to avoid affecting the evaluation of the user for the physical condition of the user due to mis-determining.

It can be learned from FIG. 7A and FIG. 7B that the electronic device 100 may simultaneously display blood pressure measurement results obtained when the user performs a plurality of measurements on the blood pressure, so that the user learns a change of the blood pressure of the user in a period of time, to help the user adjust the physical condition of the user in time, and maintain normal blood pressure fluctuations.

It should be understood that FIG. 1A to FIG. 1J, FIG. 2, FIG. 3, FIG. 4, FIG. 5, FIG. 6, and FIG. 7A and FIG. 7B are user interfaces described in detail by using an example in which whether the user has the irregular heartbeat symptom is measured. In another embodiment of this application, the irregular heartbeat may be replaced with another similar expression, for example, an abnormal heartbeat, arrhythmia, atrial fibrillation, or an irregular heart rate. In addition, the high-pressure mode and the low-pressure mode in this specification may also be replaced with other expressions. For example, the high-pressure mode is replaced with an atrial fibrillation mode, and the low-pressure mode is replaced with a non-atrial fibrillation mode; or the high-pressure mode is replaced with a special mode, and the low-pressure mode is replaced with a common mode. These similar names and expressions are not limited in embodiments of this application.

**FIG. 8** **is an overall schematic flowchart of a blood pressure measurement method according to an embodiment of this application.**

As shown in FIG. 8, the blood pressure measurement method may include but is not limited to the following steps.

S101: An electronic device 100 detects a blood pressure measurement operation.

The electronic device 100 may be a non-wearable device like a mobile phone, a tablet, or a computer, or may be a wearable device like a watch, a band, a ring, or glasses. In embodiments of this application, a device type of the electronic device 100 is not limited.

The operation may be a touch operation performed on the display, or may be a voice instruction of the user, or the like. An operation type of the operation is not limited in embodiments of this application.

For example, before the electronic device 100 detects the blood pressure measurement operation, the electronic device 100 may display a user interface including a blood pressure measurement option, and the blood pressure measurement operation may be a user operation performed on the blood pressure measurement option.

As shown in FIG. 1A, the operation may be a user operation performed on the blood pressure measurement option 211.

S102: The electronic device 100 determines, based on historically collected first data and/or second data collected after the blood pressure measurement operation is detected, whether a user has an irregular heartbeat symptom.

The first data may be data in duration before the blood pressure measurement operation of the user is currently detected. For example, the duration may be 30 minutes. The duration is not limited in embodiments of this application.

To be specific, that the electronic device 100 determines, based on the historical data of the user, whether the user has the irregular heartbeat symptom means that the electronic device 100 identifies whether the user has had the irregular heartbeat symptom in a recent period of time.

The electronic device 100 may determine, by recognizing whether the first data fluctuates regularly with time, whether the user has the irregular heartbeat symptom. If the first data fluctuates regularly with time, the user does not have the irregular heartbeat symptom. If the first data fluctuates irregularly with time, the user has the irregular heartbeat symptom.

In an implementation, the electronic device 100 may identify, by inputting the first data into a first preset model, whether the user has the irregular heartbeat symptom. The first preset model may be a model obtained by performing training on the first data of a user having a regular heartbeat and the first data of a user having an irregular heartbeat.

It may be understood that in another manner, the electronic device 100 may further determine, based on the first data, whether the user has the irregular heartbeat symptom. For example, the electronic device 100 may extract a data feature of the first data, where the data feature indicates a change trend of the data, and identify, by comparing a difference between the data feature of the first data and a threshold, whether the user has the irregular heartbeat symptom. An implementation of determining, based on the first data, whether the user has the irregular heartbeat symptom is not limited in embodiments of this application.

For example, the first data may be a PPG signal. The PPG signal may be collected by using a photoelectric sensor on the wearable device.

In this case, the first data may have the following two cases.
(1) If the electronic device 100 is the wearable device, the first data may be data collected by the electronic device 100.

In other words, the electronic device 100 may continuously collect the first data, and locally store the first data. After detecting the operation user of measuring the blood pressure by the user, the electronic device 100 may use the first data collected within preset duration before the operation, to analyze whether the user has the irregular heartbeat symptom.

(2) If the electronic device 100 is the non-wearable device, the first data may be data obtained by the electronic device 100 from the wearable device worn by the user.

In other words, after the electronic device 100 detects the blood pressure measurement operation of the user, the electronic device 100 may obtain, from the wearable device worn by the user, the first data historically collected by the wearable device, and analyze, based on the first data, whether the user has the irregular heartbeat symptom.

For example, after detecting the blood pressure measurement operation of the user, the electronic device 100 may send a message to the wearable device worn by the user, where the message is used to request to obtain the historical first data of the user.

When the electronic device 100 determines, based on the historically collected first data, whether the user has the irregular heartbeat symptom, if the electronic device 100 determines that the user does not have the irregular heartbeat symptom, the electronic device 100 may start one blood pressure measurement in response to the blood pressure measurement operation of the user, and determine a blood pressure value obtained through the one measurement as a blood pressure value of the user; or if the electronic device 100 determines that the user has the irregular heartbeat symptom, the electronic device 100 may start a plurality of blood pressure measurements in response to the blood pressure measurement operation of the user, and determine a blood pressure value of the user based on blood pressure values obtained through the plurality of measurements.

A blood pressure measurement mode of the one blood pressure measurement may be a low-pressure mode, and a blood pressure measurement mode of the plurality of blood pressure measurements may be a high-pressure mode.

The second data may be data collected in duration after the blood pressure measurement operation is detected. For example, the duration may be 5 minutes. The duration is not limited in embodiments of this application.

To be specific, that the electronic device 100 determines, based on the second data, whether the user is in an irregular heartbeat state means that the electronic device 100 identifies whether the user has the irregular heartbeat symptom after the blood pressure measurement operation is initiated.

The electronic device 100 may determine, by recognizing whether the second data fluctuates regularly with time, whether the user has the irregular heartbeat symptom. If the second data fluctuates regularly with time, the user does not have the irregular heartbeat symptom. If the second data fluctuates irregularly with time, the user has the irregular heartbeat symptom.

In an implementation, the electronic device 100 may identify, by inputting the second data into a second preset model, whether the user has the irregular heartbeat symptom. The first preset model may be a model obtained by performing training on the second data of a user having a regular heartbeat and the second data of a user having an irregular heartbeat.

It may be understood that in another manner, the electronic device 100 may further determine, based on the second data, whether the user has the irregular heartbeat symptom. For example, the electronic device 100 may extract a data feature of the second data, where the data feature indicates a change trend of the data, and identify, by comparing a difference between the data feature of the second data and a threshold, whether the user has the irregular heartbeat symptom. An implementation of determining, based on the second data, whether the user has the irregular heartbeat symptom is not limited in embodiments of this application.

Further, after detecting the blood pressure measurement operation, the electronic device 100 may start the blood pressure measurement in response to the operation, and the second data may be data collected during the blood pressure measurement. In this way, the electronic device 100 may calculate the blood pressure of the user based on the second data, and analyze, based on the second data, whether the user has the irregular heartbeat symptom. In other words, the electronic device 100 may identify, during the blood pressure measurement, whether the user has the irregular heartbeat symptom.

In this case, after the electronic device 100 detects the blood pressure measurement operation of the user, the electronic device 100 may display a corresponding user interface during the blood pressure measurement, for example, FIG. 1B and FIG. 1C, to inform the user that the blood pressure measurement is started currently.

The corresponding user interface during the blood pressure measurement may be used to display related information during the blood pressure measurement, including but not limited to a correct measurement posture, measurement duration, a pressure value during the measurement, and the like.

For example, the second data may be a PPG signal, or may be an oscillation wave signal. For specific descriptions of the oscillation wave signal, refer to the foregoing related descriptions in the principle of measuring the blood pressure by the electronic device 100. Details are not described herein again.

Similar to the first data, the second data may also have the following two cases.
(1) If the electronic device 100 is the wearable device, the second data may be data collected by the electronic device 100.

In other words, after the electronic device 100 detects the blood pressure measurement operation of the user, the electronic device 100 may analyze, based on the second data collected by the electronic device 100 in real time, whether the user has the irregular heartbeat symptom.

It may be understood that the electronic device 100 may start to collect the second data after detecting the blood pressure measurement operation of the user, and does not need to collect the second data before detecting the blood pressure measurement operation of the user. In this way, the electronic device 100 does not need to be in a second data collection state in real time, but starts to collect the second data when the user needs to measure the blood pressure, to reduce a workload of the electronic device 100.

(2) If the electronic device 100 is the non-wearable device, the second data may be data obtained by the electronic device 100 from the wearable device worn by the user.

In other words, after the electronic device 100 detects the blood pressure measurement operation, the electronic device 100 may obtain, from the wearable device worn by the user, the second data collected by the wearable device in real time, and analyze, based on the second data, whether the user has the irregular heartbeat symptom.

For example, after detecting the blood pressure measurement operation, the electronic device 100 may send a message to the wearable device worn by the user, where the message is used to request to obtain the second data of the user.

If the electronic device 100 determines, based on the second data, whether the user has the irregular heartbeat symptom, the electronic device 100 may directly start a 1^{st} blood pressure measurement after detecting the blood pressure measurement operation of the user, and identify, based on data collected during the blood pressure measurement, whether the user has the irregular heartbeat symptom. If the electronic device 100 determines that the user does not have the irregular heartbeat symptom, the electronic device 100 may directly determine a blood pressure value obtained through the blood pressure measurement as a blood pressure value of the user. If the electronic device 100 determines that the user has the irregular heartbeat symptom, after the blood pressure measurement ends, the electronic device 100 further needs to start N blood pressure measurements, and then determine a blood pressure value of the user based on blood pressure values obtained through the N blood pressure measurements, or determine the blood pressure value of the user based on the blood pressure value obtained through the 1^{st} blood pressure measurement and the blood pressure values obtained through the N blood pressure measurements.

If the blood pressure value of the user is determined based on the blood pressure values obtained through the N blood pressure measurements, N is a positive integer greater than or equal to 2. If the blood pressure value of the user is determined based on the blood pressure value obtained through the 1^{st} blood pressure measurement and the blood pressure values obtained through the N blood pressure measurements, N is a positive integer greater than or equal to 1.

When the 1^{st} blood pressure measurement is started, the blood pressure measurement mode may be the low-pressure mode, and the blood pressure measurement mode of the N blood pressure measurements may be the high-pressure mode.

Further, after the 1^{st} blood pressure measurement is started, if the electronic device 100 determines, during the 1^{st} blood pressure measurement, that the user has the irregular heartbeat symptom, the electronic device 100 may switch the blood pressure measurement mode of the 1^{st} blood pressure measurement from the low-pressure mode to the high-pressure mode, so that the blood pressure value obtained by the electronic device 100 in the 1^{st} blood pressure measurement is also a blood pressure value obtained in the high-pressure mode.

In addition to different quantities of blood pressure measurements, the low-pressure mode and the high-pressure mode may further have any one or more of the following differences.
(1) If the blood pressure measurement device is a wearable device configured with an airbag, a pressure applied when the airbag is inflated in the low-pressure mode may be lower than a pressure applied when the airbag is inflated in the high-pressure mode.

In this way, the electronic device 100 can collect a more accurate oscillation wave signal in the high-pressure mode.

Optionally, a deflation speed of the airbag in the low-pressure mode may be greater than a deflation speed of the airbag in the high-pressure mode.

In this way, discomfort of the user in the blood pressure measurement in the high-pressure mode can be reduced.

(2) A blood pressure measurement mode (referred to as a model 1 below) used in the low-pressure mode is not a model established for people having the irregular heartbeat, and a model (referred to as a model 2 below) used in the high-pressure mode is the model established for people having the irregular heartbeat.

The blood pressure measurement model is used to determine a blood pressure value of a measurement. After each blood pressure measurement, the electronic device 100 may input blood pressure data collected during the blood pressure measurement, for example, a PPG signal or an oscillation wave signal, into the blood pressure measurement model, to obtain a blood pressure value obtained through the blood pressure measurement.

It is considered that if the user has the irregular heartbeat symptom, a blood pressure value calculated by using a conventional blood pressure measurement model may not be accurate.

For example, Table 1 shows a comparison of effects of determining a blood pressure value by using different algorithms.

**Table 1**

| Test data set | Number of people | Mean error (mean error, ME) | Standard deviation (standard deviation, STD) | Error proportion (≤ 5 mmHg) | Error proportion (≤ 10 mmHg) | Error proportion (≤ 15 mmHg) |
|---|---|---|---|---|---|---|
| Conventional algorithm | 556 | 0.65 | 13.37 | 31.0% | 57.1% | 76.5% |
| High-pressure mode algorithm | 556 | 1.03 | 11.89 | 35.2% | 66.0% | 83.9% |

All subjects in the test data set have the irregular heartbeat symptom. The conventional algorithm is a method of measuring the blood pressure by using the model 1. The algorithm is an algorithm trained for healthy people. The high-pressure mode algorithm is a method of measuring the blood pressure by using the model 2. The algorithm is an algorithm trained for the people having the irregular heartbeat. The mean error is a mean error between a blood pressure value of the subject in the test data set obtained through the measurement by using an algorithm and an actual blood pressure value of the subject. The standard deviation is a standard deviation between the blood pressure value of the subject in the test data set obtained through the measurement by using the algorithm and an actual blood pressure value of the subject. The error proportion (≤ 5 mmHg) means a proportion of subjects in the test data set in total subjects in terms of a difference between a blood pressure value obtained through the measurement by using the algorithm and an actual blood pressure value of the subject being less than 5 mmHg. The error proportion (≤ 10 mmHg) means a proportion of subjects in the test data set in total subjects in terms of a difference between a blood pressure value obtained through the measurement by using the algorithm and an actual blood pressure value of the subject being less than 10 mmHg. The error proportion (≤ 15 mmHg) means a proportion of subjects in the test data set in total subjects in terms of a difference between a blood pressure value obtained through the measurement by using the algorithm and an actual blood pressure value of the subject being less than 15 mmHg.

It can be learned from Table 1 that accuracy of measuring the blood pressure value of the user having the irregular heartbeat by using the model 2 is higher than the accuracy of measuring the blood pressure value of the user having the irregular heartbeat by using the model 1.

Therefore, when measuring the blood pressure, the electronic device 100 may adopt a more targeted blood pressure measurement model based on a physical condition of the user, to obtain a more accurate blood pressure value through the measurement.

In conclusion, the electronic device 100 may determine, in any one of the following manners, whether the user has the irregular heartbeat symptom.
(1) Determine, based on the historically collected first data, whether the user has the irregular heartbeat symptom.

In this way, after detecting the blood pressure measurement operation of the user, the electronic device 100 may quickly identify, based on the historical data, whether the user has historically had the irregular heartbeat symptom.

(2) Determine, based on the second data collected after the blood pressure measurement operation is detected, whether the user has the irregular heartbeat symptom.

In this way, before the blood pressure measurement operation of the user is detected, whether the historical data of the user is collected does not need to be limited, and the electronic device 100 may determine, based on the real-time data collected after the blood pressure measurement operation of the user is detected, whether the user has the irregular heartbeat symptom after the blood pressure measurement is initiated.

(3) Determine, based on the historically collected first data and the second data collected after the blood pressure measurement operation is detected, whether the user has the irregular heartbeat symptom.

If the irregular heartbeat symptom of the user is not identified based on the first data, and the irregular heartbeat symptom of the user is not identified based on the second data, it may be determined that the user does not have the irregular heartbeat symptom. If the irregular heartbeat symptom of the user is identified based on the first data, or the irregular heartbeat symptom of the user is identified based on the second data, it may be determined that the user has the irregular heartbeat symptom.

In this way, the electronic device 100 may identify, based on the historical first data of the user and the second data collected after the blood pressure measurement operation of the user is detected, whether the user has the irregular heartbeat symptom in a period of time before and a period of time after the blood pressure measurement is initiated, to improve recognition accuracy.

If the electronic device 100 determines that the user has the irregular heartbeat symptom, step S103 may be performed, to determine the blood pressure value of the user based on blood pressure values of a plurality of measurements. Otherwise, the electronic device 100 may determine the blood pressure value of one measurement as the blood pressure value of the user.

S103: The electronic device 100 determines the blood pressure value of the user based on the blood pressure values of the plurality of measurements.

When the user has the irregular heartbeat, the blood pressure of the user also fluctuates irregularly. Consequently, the blood pressure value obtained through the measurement has a large random error. In this case, if only the blood pressure value of the one measurement is used as the blood pressure value of the user, the blood pressure value may not represent an actual blood pressure value of the user. Therefore, after determining that the user has the irregular heartbeat symptom, the electronic device 100 may determine the blood pressure value of the user based on the blood pressure values of the plurality of measurements.

In this embodiment of this application, the plurality of times may be three times. In another embodiment of this application, the plurality of times may be two times, four times, or another quantity of times. A specific quantity of the plurality of times is not limited in embodiments of this application.

The blood pressure value of the user may be an average of blood pressure values of the plurality of measurements.

Considering that when the heartbeat of the user is irregular, the blood pressure of the user also changes irregularly, if only the blood pressure value of the one measurement is used as the blood pressure value of the user, a large random error may be caused, and there is a large difference between the measured blood pressure value and the actual blood pressure value of the user.

For example, Table 2 shows a comparison of effects of directly using the result of the blood pressure measurement as the blood pressure value of the user and using the average of the results of the plurality of blood pressure measurements as the blood pressure value of the user.

**Table 2**

| Test data set | Number of people | Mean error (mean error, ME) | Standard deviation (standard deviation, STD) | Error proportion (≤ 5 mmHg) | Error proportion (≤ 10 mmHg) | Error proportion (≤ 15 mmHg) |
|---|---|---|---|---|---|---|
| Direct calculation | 556 | 1.03 | 11.89 | 35.2% | 66.0% | 83.9% |
| 1^{st} measurement | 556 | 1.23 | 12.46 | 35.9% | 68.0% | 83.0% |
| 2^{nd} measurement | 556 | 0.45 | 11.18 | 34.4% | 63.8% | 84.9% |
| Average the two measurements | 556 | -1.00 | 11.10 | 39.0% | 70.6% | 86.7% |

All the subjects in the test data set have the irregular heartbeat symptom. Direct calculation means directly performing one blood pressure measurement on the test data set, to obtain a blood pressure value. Averaging the two measurements means averaging two blood pressure values obtained by performing the 1^{st} blood pressure measurement and the 2^{nd} blood pressure measurement on the test data set, to obtain a blood pressure value. The mean error is a mean error between a blood pressure value of the subject in the test data set obtained through the measurement and an actual blood pressure value of the subject. The standard deviation is a standard deviation between a blood pressure value of the subject in the test data set obtained through the measurement and an actual blood pressure value of the subject. The error proportion (≤ 5 mmHg) means a proportion of subjects in the test data set in total subjects in terms of a difference between a blood pressure value obtained through the measurement and an actual blood pressure value of the subject being less than 5 mmHg. The error proportion (≤ 10 mmHg) means a proportion of subjects in the test data set in total subjects in terms of a difference between a blood pressure value obtained through the measurement and an actual blood pressure value of the subject being less than 10 mmHg. The error proportion (≤ 15 mmHg) means a proportion of subjects in the test data set in total subjects in terms of a difference between a blood pressure value obtained through the measurement and an actual blood pressure value of the subject being less than 15 mmHg.

It can be learned from Table 2 that, compared with directly using the blood pressure value obtained through the one measurement as the blood pressure value of the user, using the average of the blood pressure values obtained through the plurality of measurements as the blood pressure value of the user is more accurate.

It may be understood that the electronic device 100 may further calculate the blood pressure value of the user from the blood pressure values of the plurality of measurements in another calculation manner. For example, the blood pressure value of the user may be a median of the blood pressure values of the plurality of measurements. A calculation manner of determining the blood pressure value of the user based on the blood pressure values of the plurality of measurements is not limited in embodiments of this application.

In some implementations, the electronic device 100 may determine whether the electronic device 100 is in a guest mode, to select to be based on the historically collected first data or the second data collected after the blood pressure measurement operation is detected, to determine whether the user has the irregular heartbeat symptom.

If the electronic device 100 is in the guest mode, the electronic device 100 determines, based on the second data collected after the blood pressure measurement operation is detected, whether the user has the irregular heartbeat symptom. If the electronic device 100 is not in the guest mode, that is, in a non-guest mode, the electronic device 100 determines, based on the historically collected first data or further with reference to the second data collected after the blood pressure measurement operation is detected, whether the user has the irregular heartbeat symptom.

Division between the guest mode and the non-guest mode is to determine whether this is the 1^{st} time for the user who measures the blood pressure this time to measure the blood pressure by using the electronic device 100.

If this is the first time for the user to measure the blood pressure by using the electronic device 100, the electronic device 100 cannot obtain the historical data of the user. Therefore, the electronic device 100 may measure the blood pressure of the user in the guest mode, that is, determine, based on the second data collected after the blood pressure measurement operation is detected, whether the user has the irregular heartbeat symptom, and then determine the blood pressure value of the user based on the blood pressure value of the one measurement or the blood pressure values of the plurality of measurements in different cases. If this is not the first time for the user to measure the blood pressure by using the electronic device 100, the electronic device 100 can obtain the historical data of the user. Therefore, the electronic device 100 may measure the blood pressure of the user in the non-guest mode, that is, determine, based on the historically collected second data or further with reference to the second data collected after the blood pressure measurement operation is detected, whether the user has the irregular heartbeat symptom, and then determine the blood pressure value of the user based on the blood pressure value of the one measurement or the blood pressure values of the plurality of measurements in different cases.

For example, the electronic device 100 may select the guest mode or the non-guest mode in any one of the following manners.
(1) The electronic device 100 selects the guest mode or the non-guest mode based on a user operation.

Before or after detecting the blood pressure measurement operation of the user, the electronic device 100 may display an option corresponding to the guest mode and an option corresponding to the non-guest mode. If the electronic device 100 detects a selection operation performed on the option corresponding to the guest mode, the guest mode is selected. If the electronic device 100 detects a selection operation performed on the option corresponding to the non-guest mode, the non-guest mode is selected.

In other words, the electronic device 100 may select, based on the user operation during the blood pressure measurement, whether to identify the irregular heartbeat symptom of the user based on the historically collected first data or the second data collected after the blood pressure measurement operation is detected.

(2) The electronic device 100 automatically selects the guest mode or the non-guest mode based on physiological information of the user.

The physiological information may include a body action, a breathing frequency, a shell temperature, a fingerprint, an iris, and the like of the user. The electronic device 100 may determine, based on a change of the collected physiological information, whether the user who measures the blood pressure changes, to implement selection between the guest mode and the non-guest mode.

For example, after the user initiates the blood pressure measurement, if the electronic device 100 detects that physiological indicators such as a breathing frequency and a shell temperature of the user are different from physiological indicators during the previous blood pressure measurement, it indicates that the user who initiates the blood pressure measurement this time is different from the user who measures the blood pressure previously. Therefore, the electronic device 100 may select the guest mode.

For another example, related data may include a wrist circumference. For example, the electronic device 100 is the wearable device. The electronic device 100 may select the guest mode or the non-guest mode based on a change of the wrist circumference. Because wrists of different users are of different thicknesses, when the electronic device 100 detects that the wrist circumference of the user during the blood pressure measurement is different from the wrist circumference during the previous blood pressure measurement, it indicates that the user who initiates the blood pressure measurement this time is different from the user who measures the blood pressure previously. Therefore, the electronic device 100 may select the guest mode. If the wrist circumference of the user is the same as the wrist circumference during the previous blood pressure measurement, it indicates that the user who initiates the blood pressure measurement this time is the user who measures the blood pressure previously. Therefore, the electronic device 100 may select the non-guest mode.

It may be understood that the electronic device 100 may further implement selection between the guest mode and the non-guest mode in another manner. This is not limited in embodiments of this application.

For example, the electronic device 100 may determine, when the user wears the wearable device used for the blood pressure measurement, whether the electronic device 100 is in the guest mode; or the electronic device 100 may determine, before the first data is collected in the background, whether the electronic device 100 is in the guest mode; or the electronic device 100 may determine, after the user blood pressure measurement operation is detected, whether the electronic device 100 is in the guest mode. An execution occasion at which the electronic device 100 determines whether the electronic device 100 is in the guest mode is not limited in embodiments of this application.

In some implementations, the electronic device 100 may display a result of one blood pressure measurement or results of a plurality of blood pressure measurements. The blood pressure measurement result may include the blood pressure value of the user measured by the electronic device 100. The user may learn a blood pressure status of the user based on the blood pressure value. Further, the user may learn a change of the blood pressure of the user based on the blood pressure values of the plurality of measurements.

Further, the blood pressure measurement result may further include a measurement identifier, and the measurement identifier may indicate a measurement state of the user. For example, the measurement identifier may indicate that the user does not perform the measurement in a specified posture, or the measurement identifier may indicate that the blood pressure value is calculated through the plurality of blood pressure measurements after it is identified that the user has the irregular heartbeat symptom, or the measurement identifier may indicate that the body of the user moves or does not stay stationary during the measurement, or the measurement identifier may indicate that the user performs exercise before the measurement.

For example, FIG. 7A shows the user interface 37 in which the electronic device 100 displays the measurement result. The user interface 37 includes the measurement identifier 371A, the measurement identifier 372A, the measurement identifier 373A, and the measurement identifier 374A. The measurement identifier 371A may indicate that the user does not perform the measurement in a correct posture during the measurement. The measurement identifier 372A may indicate that a measurement process is performed in the high-pressure mode. The measurement identifier 373A may indicate that the body of the user moves or does not stay stationary during the measurement. The measurement identifier 374A may indicate that the blood pressure measurement is performed after the user exercises.

In this way, based on the measurement identifier, the user can more clearly learn a physical condition of the user before or in the measurement, and whether the measurement result is reliable is reflected from the side.

In some implementations, because the electronic device 100 may continuously obtain heartbeat data of the user, for example, the PPG signal, the electronic device 100 may monitor a heartbeat status of the user for a long time, and summarize whether the user has the irregular heartbeat. If the electronic device 100 does not identify the irregular heartbeat symptom based on the PPG signal of the user for a long time, for example, within 10 days, the electronic device 100 may directly determine that the user has no possibility of the irregular heartbeat. In this way, when the blood pressure measurement operation of the user is subsequently detected, there is no need to determine whether the user has the irregular heartbeat symptom, and a blood pressure measurement manner for normal people is directly started, namely, the blood pressure measurement in the low-pressure mode, to reduce determining logic, so as to save operation resources.

In some implementations, in the blood pressure values of the plurality of measurements, a difference between blood pressure values of any two measurements is less than a first threshold, and/or a heart rate value of the user during each measurement is less than a second threshold, and/or a heart rate difference of the user during any two measurements is less than a third threshold.

For example, the first threshold may be 10, the second threshold may be 90, and the third threshold may be 10.

If a difference between blood pressure values of two measurements is excessively large, or a heart rate value of the user during the measurement is excessively large, or a difference between heart rate values of the user during two measurements is excessively large, it indicates that an error of a result of one blood pressure measurement is large. Therefore, the electronic device 100 may discard the measurement result with a large error, to avoid interference of the measurement result in calculation of the blood pressure value of the user.

In other words, a quantity of actual measurements of the electronic device 100 may be greater than a quantity of measurements finally used for determining the blood pressure value of the user. The electronic device 100 may identify, during the plurality of blood pressure measurements, whether an error of the measurement result is excessively large, to prompt, in time, the user to measure the blood pressure again. For example, FIG. 4 is the user interface 34 displayed by the electronic device 100 when it is identified that there is a measurement result with a large error.

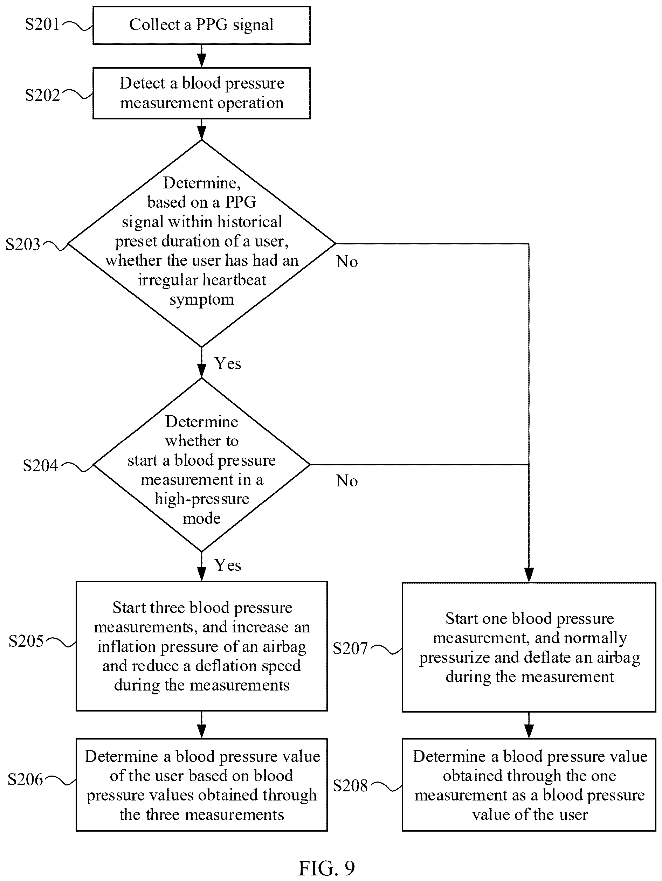

**FIG. 9** **is a schematic flowchart of measuring, by an electronic device 100, a blood pressure in a non-guest mode according to an embodiment of this application.**

S201: The electronic device 100 collects a PPG signal.

The electronic device 100 may be a wearable device like a watch or a band. In addition, an airbag is configured on a strap of the electronic device 100. When starting a blood pressure measurement, the electronic device 100 may inflate the airbag to block arterial blood flow, collect an oscillation wave signal in an inflation process, and estimate a blood pressure value based on the oscillation wave signal.

In addition, in a process in which a user wears the electronic device 100, the electronic device 100 may continuously collect the PPG signal.

In some implementations, before performing step S201, the electronic device 100 may determine whether the electronic device 100 is in a guest mode, and perform step S201 when the electronic device 100 is not in the guest mode, that is, in a non-guest mode.

For example, the electronic device 100 may determine, based on a user operation, whether the electronic device 100 is in the guest mode. For example, if the electronic device 100 detects a user operation performed in the non-guest mode, the electronic device 100 determines that the electronic device 100 is in the non-guest mode.

S202: The electronic device 100 detects a blood pressure measurement operation.

For example, the operation may be a touch operation performed on a display, or may be a voice instruction of the user, or the like. An operation type of the operation is not limited in embodiments of this application.

S203: The electronic device 100 determines, based on a PPG signal within historical preset duration of the user, whether the user has had an irregular heartbeat symptom.

For example, the preset duration may be 30 minutes.

The electronic device 100 may determine, based on whether a change of the PPG signal in the historical preset duration of the user is regular, whether the user has had the irregular heartbeat symptom. If the change of the PPG signal in the historical preset duration of the user is irregular, the user has had the irregular heartbeat symptom; or if the change of the PPG signal in the historical preset duration of the user is regular, the user never has the irregular heartbeat symptom.

If the user has had the irregular heartbeat symptom, the electronic device 100 may perform step S204; otherwise, the electronic device 100 may perform step S207.

S204: The electronic device 100 determines whether to start a blood pressure measurement in a high-pressure mode.

For example, the electronic device 100 may determine, based on a user operation, whether to start the blood pressure measurement in the high-pressure mode. If the electronic device 100 starts the blood pressure measurement in the high-pressure mode, step S205 is performed; otherwise, the electronic device 100 starts a blood pressure measurement in a low-pressure mode, that is, step S207 is performed.

Specifically, after the electronic device 100 determines that the user has had the irregular heartbeat symptom, the electronic device 100 may display an option corresponding to the high-pressure mode. If the electronic device 100 detects a user operation performed on the option corresponding to the high-pressure mode, the electronic device 100 starts the blood pressure measurement in the high-pressure mode, that is, performs step S205.

Correspondingly, after the electronic device 100 determines that the user has had the irregular heartbeat symptom, the electronic device 100 may further display an option corresponding to the low-pressure mode. If the electronic device 100 detects a user operation performed on the option corresponding to the low-pressure mode, the electronic device 100 starts the blood pressure measurement in the low-pressure mode, that is, performs step S207.

It may be understood that step S204 is an optional step. After determining that the user has the irregular heartbeat symptom, the electronic device 100 may directly start the blood pressure measurement in the high-pressure mode, that is, perform step S205, without a need to determine whether to start the blood pressure measurement in the high-pressure mode, to reduce inconvenience of a user operation.

S205: The electronic device 100 starts three blood pressure measurements, and increases an inflation pressure of the airbag and reduces a deflation speed during the measurements.

In the high-pressure mode, the electronic device 100 may perform a plurality of consecutive blood pressure measurements, and during each blood pressure measurement, the electronic device 100 may increase the inflation pressure of the airbag and reduce the deflation speed of the airbag.

It should be noted that, the electronic device 100 increases the inflation pressure of the airbag and reduces the deflation speed of the airbag relative to the low-pressure mode. In other words, in comparison with the low-pressure mode, in the high-pressure mode, the electronic device 100 applies a higher inflation pressure to the airbag. Optionally, when the airbag pressure is released, the deflation speed is smaller. In this way, accuracy of a measured blood pressure value can be ensured when the user has the irregular heartbeat symptom.

For example, in the process in which the electronic device 100 starts the three blood pressure measurements, the electronic device 100 may input, into a model 2, an oscillation wave signal collected during one measurement, to calculate a blood pressure value obtained through the one measurement. The model 2 is obtained by performing training on an oscillation wave signal of a subject whose blood pressure is known to be irregular.

S206: The electronic device 100 determines a blood pressure value of the user based on blood pressure values obtained through the three measurements.

For example, the electronic device 100 may determine an average of the blood pressure values obtained through the three measurements as the blood pressure value of the user.

In some implementations, in a process in which the electronic device 100 perform the plurality of consecutive blood pressure measurements, if the electronic device 100 detects that data of one of the plurality of blood pressure measurements is excessively different from data of other measurements, the data of the measurement may be incorrect. The electronic device 100 may discard the data of the measurement, perform another blood pressure measurement, and determine the blood pressure value of the user based on the data of the another measurement and the data of the other measurements with a small error.

S207: The electronic device 100 starts one blood pressure measurement, and normally pressurizes and deflates the airbag during the measurement.

In the low-pressure mode, the electronic device 100 only needs to perform one blood pressure measurement, and during the blood pressure measurement, the electronic device 100 may measure the blood pressure under the normal inflation pressure of the airbag and at a normal deflation speed.

It may be understood that the electronic device 100 normally pressurizes and deflates the airbag relative to the high-pressure mode. For specific descriptions of this, refer to related content in step S205. Details are not described herein again.

For example, in the process in which the electronic device 100 starts the one blood pressure measurement, the electronic device 100 may input, into a model 1, an oscillation wave signal collected during the measurement, to calculate a blood pressure value. The model 1 is obtained by performing training on an oscillation wave signal of a subject whose blood pressure is known to be irregular.

Optionally, after the electronic device 100 starts the one blood pressure measurement, the electronic device 100 may determine, based on second data collected during the blood pressure measurement, whether the user has the irregular heartbeat symptom. If the user does not have the irregular heartbeat symptom, the electronic device 100 may perform step S208. Otherwise, the electronic device 100 may start the blood pressure measurement in the high-pressure mode, and determine the blood pressure value of the user based on blood pressure values of the plurality of measurements. In this way, the electronic device 100 can determine, based on historical data, whether the user has the irregular heartbeat symptom, and further determine, based on the data collected during the measurement, whether the user has the irregular heartbeat symptom, to improve recognition accuracy of the symptom of the user.

For specific descriptions of determining, by the electronic device 100 during the blood pressure measurement, whether the user has the irregular heartbeat, refer to related content in step S102. Details are not described herein again.

S208: The electronic device 100 determines the blood pressure value obtained through the one measurement as the blood pressure value of the user.

Because when the user has a regular heartbeat, the electronic device 100 can reflect a blood pressure status of the user based on the blood pressure value of the one measurement, the electronic device 100 may determine the blood pressure value obtained through the one measurement as the blood pressure value of the user.

It can be learned from steps S201 to S208 that, if the electronic device 100 measures the blood pressure in the non-guest mode, the electronic device 100 may first determine, based on a historical PPG signal of the user, whether the user has the irregular heartbeat symptom. If the user has the irregular heartbeat symptom, the electronic device 100 starts the blood pressure measurement in the high-pressure mode, and calculates the blood pressure value of the user based on the blood pressure values of the plurality of measurements. If the user does not have irregular heartbeat symptom, the electronic device 100 starts the blood pressure measurement in the low-pressure mode, and determines the blood pressure value of the one measurement as the blood pressure value of the user. In this way, when the user initiates the blood pressure measurement, a physical condition of the user can be evaluated based on the historical data of the user, and a targeted blood pressure measurement mode is used to accurately measure the blood pressure of the user, to improve accuracy of the blood pressure measurement.

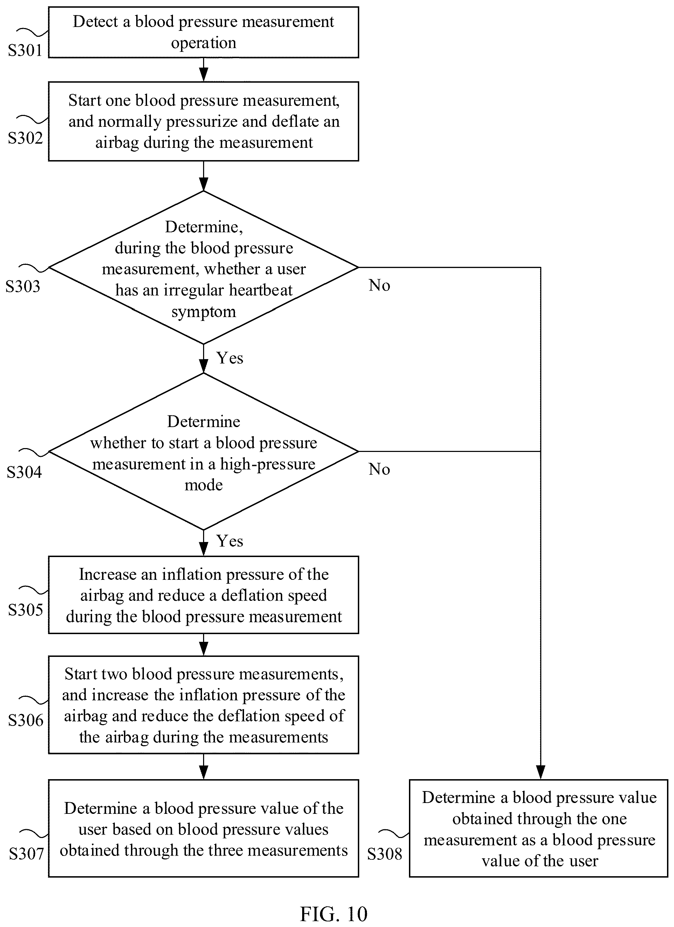

**FIG. 10** **is a schematic flowchart of measuring, by an electronic device 100, a blood pressure in a guest mode according to an embodiment of this application.**

S301: The electronic device 100 detects a blood pressure measurement operation.

In some implementations, before performing step S301, the electronic device 100 may determine whether the electronic device 100 is in a guest mode, and perform step S301 when the electronic device 100 is in the guest mode.

For example, the electronic device 100 may determine, based on a user operation, whether the electronic device 100 is in the guest mode. For example, if the electronic device 100 detects a user operation performed in the guest mode, the electronic device 100 determines that the electronic device 100 is in the guest mode.

S302: The electronic device 100 starts one blood pressure measurement, and normally pressurizes and deflates an airbag during the measurement.

The electronic device 100 may directly start a blood pressure measurement in a low-pressure mode in response to the blood pressure measurement operation of a user.

For specific descriptions of the low-pressure mode, refer to related content in step S207. Details are not described herein again.

S303: The electronic device 100 determines, during the blood pressure measurement, whether the user has an irregular heartbeat symptom.

The electronic device 100 may determine, during the blood pressure measurement based on a collected oscillation wave signal, whether the user has the irregular heartbeat symptom. If a change of the oscillation wave signal of the user is irregular, the user has the irregular heartbeat symptom. If the change of the oscillation wave signal of the user is regular, the user does not have irregular heartbeat symptom.

If the electronic device 100 determines, during the blood pressure measurement, that the user has the irregular heartbeat symptom, the electronic device 100 may perform step S304; otherwise, the electronic device 100 may perform step S308.

S304: The electronic device 100 determines whether to start a blood pressure measurement in a high-pressure mode.

For example, the electronic device 100 may determine, based on a user operation, whether to start the blood pressure measurement in the high-pressure mode. If the electronic device 100 starts the blood pressure measurement in the high-pressure mode, step S305 is performed; otherwise, step S308 is performed.

It may be understood that step S304 is an optional step. After recognizing that the user has the irregular heartbeat symptom, the electronic device 100 may directly switch to the high-pressure mode to measure a blood pressure of the user, without a need to determine whether to start the blood pressure measurement in the high-pressure mode, to reduce inconvenience of a user operation.

S305: The electronic device 100 increases an inflation pressure of the airbag and reduces a deflation speed during the blood pressure measurement.

It is considered that during the blood pressure measurement of the electronic device 100, it only needs a short time to identify, based on the oscillation wave signal collected during the blood pressure measurement, whether the user has the irregular heartbeat symptom. If the electronic device 100 identifies that the user has the irregular heartbeat symptom, a blood pressure measurement mode of the blood pressure measurement may be switched from the low-pressure mode to the high-pressure mode. Specifically, it may be represented as increasing the inflation speed of the airbag, and optionally, reducing the deflation speed during the measurement.

It may be understood that step S305 is an optional step, and the electronic device 100 may continue to use the low-pressure mode during a 1^{st} blood pressure measurement, and change the blood pressure measurement mode in a subsequent blood pressure measurement to the high-pressure mode.

S306: The electronic device 100 starts two blood pressure measurements, and increases the inflation pressure of the airbag and reduces the deflation speed during the measurements.

After the 1^{st} blood pressure measurement mentioned in step S302 ends, the electronic device 100 may start the two blood pressure measurements, and the blood pressure measurement mode of the two blood pressure measurements is the high-pressure mode. Specifically, an inflation pressure of the airbag in the high-pressure mode is higher than an inflation pressure of the airbag in the low-pressure mode. Further optionally, a deflation speed of the airbag in the high-pressure mode is less than a deflation speed of the airbag in the low-pressure mode.

It may be understood that increasing the inflation pressure and reducing the deflation speed are relative to the inflation pressure and the deflation speed in the low-pressure mode. For specific related descriptions of the blood pressure measurement in the high-pressure mode, refer to related content in step S205. Details are not described herein again.

S307: The electronic device 100 determines a blood pressure value of the user based on blood pressure values obtained through three measurements.

For example, the electronic device 100 may determine an average of the blood pressure values obtained through the three measurements as the blood pressure value of the user.

In addition, for an oscillation wave signal collected during each measurement, the electronic device 100 may calculate, by using a model 2, the blood pressure value obtained through the measurement.

S308: The electronic device 100 determines a blood pressure value obtained through one measurement as the blood pressure value of the user.

If the electronic device 100 determines that the user does not have the irregular heartbeat symptom, or does not start the blood pressure measurement in the high-pressure mode, the electronic device 100 may remain in the low-pressure mode, complete the one blood pressure measurement, and directly determine the blood pressure value obtained through the one measurement as the blood pressure value of the user.

The electronic device 100 may input, into a model 1, an oscillation wave signal collected during the measurement, to calculate the blood pressure value. The model 1 is obtained by performing training on an oscillation wave signal of a subject whose blood pressure is known to be irregular.

For specific content that is not mentioned or not described in detail in steps S301 to S308, refer to related content in steps S201 to S208. Details are not described herein again.

It can be learned from steps S301 to S308 that, if the electronic device 100 measures the blood pressure in the guest mode, the electronic device 100 may first start the blood pressure measurement in the low-pressure mode by default, and detect, during the blood pressure measurement, whether the user has the irregular heartbeat symptom. If the user has the irregular heartbeat symptom, the electronic device 100 may switch to the blood pressure measurement in the high-pressure mode, and calculate the blood pressure value of the user based on the blood pressure values of the plurality of measurements. If the user does not have the irregular heartbeat symptom, the electronic device 100 may directly determine the blood pressure value obtained through the measurement in the low-pressure mode as the blood pressure value of the user. In this way, the data collected during the blood pressure measurement may be used to evaluate a physical condition of the user, and a blood pressure measurement mode may be switched in time to perform a targeted measurement on the blood pressure of the user, to improve accuracy of the blood pressure measurement.

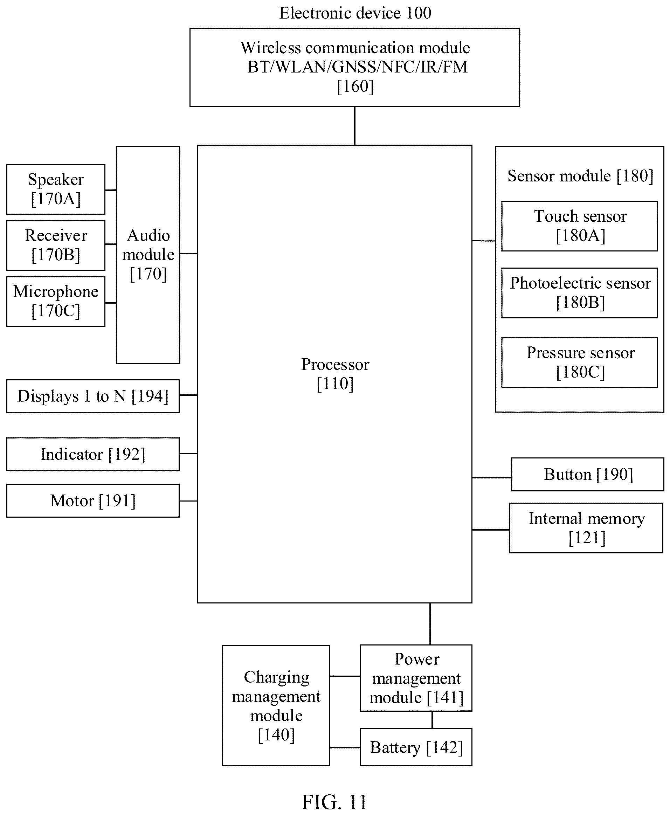

**FIG. 11** **is a diagram of a hardware structure of an electronic device 100.**

The electronic device 100 may be a mobile phone, a tablet computer, a desktop computer, a laptop computer, a handheld computer, a notebook computer, an ultra-mobile personal computer (ultra-mobile personal computer, UMPC), a netbook, a cellular phone, or a personal digital assistant (personal digital assistant, PDA), augmented reality (augmented reality, AR) device, virtual reality (virtual reality, VR) device, artificial intelligence (artificial intelligence, AI) device, wearable device, in-vehicle device, smart home device, and/or smart city device, a specific type of the electronic device is not limited in the embodiment of this application.

The electronic device 100 may include a processor 110, an internal memory 121, a charging management module 140, a power management module 141, a battery 142, a sensor module 180, a display 194, and the like. Optionally, the electronic device 100 may further include any one or more of a wireless communication module 160, an audio module 170, a button 190, a motor 191, an indicator 192, an airbag, and the like. The audio module 170 may include any one or more of a speaker 170A, a receiver 170B, and a microphone 170C. The sensor module 180 may include a touch sensor 180A, a photoelectric sensor 180B, a pressure sensor 180C, and the like. The airbag may be inflated and deflated during a blood pressure measurement, to block subcutaneous arterial blood flow, so that the electronic device 100 collects, through the pressure sensor 180C, data used for blood pressure calculation. For example, if the electronic device 100 is a wearable device like a watch or a band, the airbag may be disposed on a strap of the electronic device 100.

It may be understood that the structure shown in embodiments of the present invention does not constitute a specific limitation on the electronic device 100. In some other embodiments of this application, the electronic device 100 may include more or fewer components than those shown in the figure, or a combination of a part of the components, or splits from a part of the components, or an arrangement of different components. The components shown in the figure may be implemented by hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, and/or a neural-network processing unit (neural-network processing unit, NPU). Different processing units may be independent components, or may be integrated into one or more processors.

The controller may generate an operation control signal based on instruction operation code and a time sequence signal, to complete control of instruction reading and instruction execution.

A memory may be further disposed in the processor 110, and is configured to store instructions and data. In some embodiments, the memory in the processor 110 is a cache. The memory may store instructions or data just used or cyclically used by the processor 110. If the processor 110 needs to use the instruction or the data again, the processor may directly invoke the instruction or the data from the memory. This avoids repeated access, reduces a waiting time of the processor 110, and improves system efficiency.

In some implementations, the processor 110 may determine, based on historically collected first data and/or second data collected after a blood pressure measurement operation is detected, whether a user has an irregular heartbeat symptom, and when the user has the irregular heartbeat symptom, determine a blood pressure value of the user based on blood pressure values of a plurality of measurements.

In some embodiments, the processor 110 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like.

The charging management module 140 is configured to receive a charging input from the charger. The charger may be a wireless charger or a wired charger. In some embodiments of wired charging, the charging management module 140 may receive a charging input of a wired charger. In some embodiments of wireless charging, the charging management module 140 may receive a wireless charging input through a wireless charging coil of the electronic device 100. The charging management module 140 may further supply power to the electronic device through the power management module 141 while charging the battery 142.

The power management module 141 is configured to connect the battery 142, the charging management module 140, and the processor 110. The power management module 141 receives an input from the battery 142 and/or the charging management module 140, and supplies power to the processor 110, the internal memory 121, the display 194, the wireless communication module 160, and the like. The power management module 141 may be further configured to monitor parameters such as a battery capacity, a battery cycle count, and a battery health status (electric leakage or impedance). In some other embodiments, the power management module 141 may alternatively be disposed in the processor 110. In some other embodiments, the power management module 141 and the charging management module 140 may alternatively be disposed in a same device.

The wireless communication module 160 may provide a wireless communication solution that is applied to the electronic device 100 and that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, a near link (NearLink), intra-body communication (intrabodycommunication, IBC), or the like. For example, when two electronic devices communicate with each other by using an intra-body communication solution, the two electronic devices each have at least one electrode in contact with skin, and the two electronic devices receive and send information to each other through a human body by using the electrode in contact with the skin. The wireless communication module 160 may be one or more components integrating at least one communication processor module.

The electronic device 100 may implement a display function through the GPU, the display 194, the application processor, and the like. The GPU is a microprocessor for image processing, and is connected to the display 194 and the application processor. The GPU is configured to: perform mathematical and geometric computation, and render an image. The processor 110 may include one or more GPUs, which execute program instructions to generate or change display information.

The display 194 is configured to display an image, a video, and the like. The display 194 includes a display panel. The display panel may be a liquid crystal display (liquid crystal display, LCD), and the display panel may alternatively be manufactured by an organic light-emitting diode (organic light-emitting diode, OLED), an active-matrix organic light-emitting diode (active-matrix organic light-emitting diode, AMOLED), a flexible light-emitting diode (flexible light-emitting diode, FLED), a mini-LED, a micro-LED, a micro-OLED, a quantum dot light-emitting diode (quantum dot light-emitting diode, QLED), or the like. In some embodiments, the electronic device 100 may include one or N displays 194, where N is a positive integer greater than 1.

In some implementations, the display 194 may be configured to display a related user interface during the blood pressure measurement, and a blood pressure value of the user obtained after blood pressure measurement ends. For specific content displayed on the display 194, refer to related content in FIG. 1A to FIG. 1J, FIG. 2, FIG. 3, FIG. 4, FIG. 5, FIG. 6, FIG. 7A, and FIG. 7B.

The internal memory 121 may include one or more random access memories (random access memory, RAM) and one or more non-volatile memories (non-volatile memory, NVM). The random access memory may be directly read and written by using the processor 110. The random access memory may be configured to store an executable program (for example, machine instructions) in an operating system or another running program, and may be further configured to store data of a user, data of an application, and the like. The nonvolatile memory may also store an executable program, data of a user, data of an application, and the like, which may be loaded into the random access memory in advance for directly reading and writing by using the processor 110.

In some implementations, the internal memory 121 may be configured to store the historically collected first data, the second data collected after the blood pressure measurement operation is detected, the blood pressure value obtained through the measurement, the blood pressure value of the user determined based on the measured blood pressure value, and the like.

The electronic device 100 may implement an audio function, for example, music playing and recording, through the audio module 170, the speaker 170A, the receiver 170B, the microphone 170C, the application processor, and the like.

The audio module 170 is configured to convert digital audio information into an analog audio signal for output, and is further configured to convert an analog audio input into a digital audio signal. The audio module 170 may be further configured to encode and decode an audio signal. In some embodiments, the audio module 170 may be disposed in the processor 110, or some functional modules in the audio module 170 are disposed in the processor 110.

The speaker 170A, also referred to as a "loudspeaker", is configured to convert an audio electrical signal into a sound signal. The electronic device 100 may be used to listen to music or answer a call in a hands-free mode through the speaker 170A.

The receiver 170B, also referred to as an "earpiece", is configured to convert an audio electrical signal into a sound signal. When a call is answered or a speech message is received through the electronic device 100, the receiver 170B may be put close to a human ear to listen to a speech.

The microphone 170C, also referred to as a "mike" or a "mic", is configured to convert a sound signal into an electrical signal. When making a call or sending a speech message, the user may place the mouth of the user near the microphone 170C to make a sound, to input a sound signal to the microphone 170C. At least one microphone 170C may be disposed in the electronic device 100. In some other embodiments, two microphones 170C may be disposed in the electronic device 100, to collect a sound signal and implement a noise reduction function. In some other embodiments, three, four, or more microphones 170C may alternatively be disposed in the electronic device 100, to collect a sound signal, implement noise reduction, and identify a sound source, so as to implement a directional recording function and the like.

The touch sensor 180A is also referred to as a "touch component". The touch sensor 180A may be disposed on the display 194, and the touch sensor 180A and the display 194 form a touchscreen, which is also referred to as a "touchscreen". The touch sensor 180A is configured to detect a touch operation performed on or near the touch sensor 180A. The touch sensor may transfer the detected touch operation to the application processor to determine a type of a touch event. A visual output related to the touch operation may be provided on the display 194. In some other embodiments, the touch sensor 180A may also be disposed on a surface of the electronic device 100 at a position different from that of the display 194.

The photoelectric sensor 180B is configured to monitor a cardiovascular vital sign. The photoelectric sensor 180B includes at least a pair of a light-emitting diode and a photoelectric detector. The light-emitting diode is used as a light source to irradiate skin. The photoelectric detector detects remaining transmission or reflected light after light is absorbed by blood and tissue in a penetration process, and converts the remaining transmission or reflected light into an electrical signal, to obtain a PPG signal. Because intensity of the transmission light or the reflected light varies with arterial pulsation, the PPG signal also follows the arterial pulsation, that is, a heartbeat of the user fluctuates rhythmically. Based on the PPG signal, parameters such as a heart rate, blood oxygen saturation, and a blood pressure of the user may be calculated, and a heartbeat status of the user is identified, to determine whether the user has the irregular heartbeat symptom.

The pressure sensor 180C is configured to sense a pressure signal, and can convert the pressure signal into an electrical signal. In some embodiments, the pressure sensor 180C may be disposed on the display 194. There are a plurality of types of pressure sensors 180C, such as a resistive pressure sensor, an inductive pressure sensor, and a capacitive pressure sensor. The capacitive pressure sensor may include at least two parallel plates made of conductive materials. When a force acts on the pressure sensor 180C, capacitance between electrodes changes. The electronic device 100 determines pressure intensity based on the change in the capacitance.

In some implementations, the pressure sensor 180C may sense, in a process in which the airbag of the electronic device 100 is inflated or deflated, a pressure pulse transferred in a process in which arterial blood flow of the user is compressed by the airbag, to obtain an oscillation wave signal, so that the electronic device 100 can calculate parameters such as a blood pressure and a heart rate of the user, and identify a heartbeat status of the user based on the oscillation wave signal, to determine whether the user has the irregular heartbeat symptom.

The button 190 includes a power button, a volume button, and the like. The button 190 may be a mechanical button, or may be a touch button. The electronic device 100 may receive a button input, and generate a button signal input related to a user setting and function control of the electronic device 100.

The motor 191 may generate a vibration prompt. The motor 191 may be configured to provide an incoming call vibration prompt and a touch vibration feedback. For example, touch operations performed on different applications (for example, image shooting and audio playing) may correspond to different vibration feedback effect. The motor 191 may also correspond to different vibration feedback effect for touch operations performed on different areas of the display 194. Different application scenarios (for example, a time reminder, information receiving, an alarm clock, and a game) may also correspond to different vibration feedback effect. Touch vibration feedback effect may also be customized.

The indicator 192 may be an indicator light, and may be configured to indicate a charging status and a state of charge change, or may be configured to indicate a message, a missed call, a notification, and the like.

In some embodiments, the sensor module 180 of the electronic device 100 may further include any one or more of the following sensors: an acceleration sensor, a barometric pressure sensor, a temperature sensor, a gyro sensor, and the like.

The acceleration sensor may detect accelerations in various directions (usually on three axes) of the electronic device 100. When the electronic device 100 is still, a magnitude and a direction of gravity may be detected. The acceleration sensor may be further configured to identify a posture of the electronic device, and is used in switching between a landscape mode and a portrait mode, a pedometer, or another application.

The barometric pressure sensor may be configured to measure a barometric pressure. In some embodiments, the barometric pressure sensor may also be configured to measure a water pressure.

The temperature sensor may be configured to measure a body temperature of the user, or may be configured to measure a temperature of an environment in which the user is located.

The gyro sensor may be configured to determine a motion posture of the electronic device 100. In some embodiments, an angular velocity of the electronic device 100 around three axes (axes x, y, and z) may be determined through the gyro sensor.

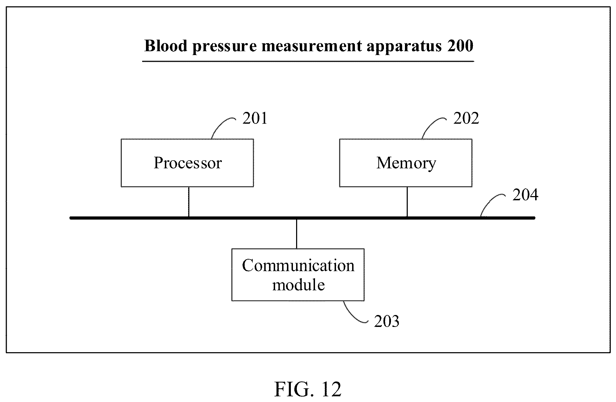

**FIG. 12** **is a diagram of a structure of a blood pressure measurement apparatus 200 according to an embodiment of this application.**

As shown in FIG. 12, the blood pressure measurement apparatus 200 may include components such as a processor 201, a memory 202, and a communication module 203. These components may be connected through a bus 204 or in another manner. In FIG. 12, an example in which the components are connected through the bus is used. The bus 204 is configured to implement communication connection between the processor 201, the memory 202, and the communication module 203.

The processor 201 may include one or more processing units. The processor 201 may be configured to provide a calculation and control capability, to support running of the entire blood pressure measurement apparatus 200.

The memory 202 may be configured to store various software programs and/or a plurality of groups of instructions. Specifically, the memory 202 may include a high-speed random access memory, or a nonvolatile memory, for example, one or more disk storage devices, a flash storage device, or another nonvolatile solid-state storage device.

The communication module 203 may be configured to communicate with another communication device. Specifically, the communication module 203 may include a communication interface. The communication interface may be a 3G communication interface, a long term evolution (LTE) (4G) communication interface, a 5G communication interface, a WLAN communication interface, a WAN communication interface, a human skin communication interface, or the like. In addition to a wireless communication interface, the blood pressure measurement apparatus 200 may be further configured with a wired communication interface to support wired communication.

In this embodiment of this application, the blood pressure measurement apparatus 200 may be the electronic device 100. The processor 201 may be configured to: after a blood pressure measurement operation is detected, determine, based on historically collected first data and/or second data collected after the blood pressure measurement operation is detected, whether a user has an irregular heartbeat symptom, and when the user has the irregular heartbeat symptom, determine a blood pressure value of the user based on blood pressure values of a plurality of measurements. The memory 202 may be configured to store the first data and/or the second data, and software or program code required by all or some functions of the electronic device 100 in the foregoing method embodiments.

It should be noted that the blood pressure measurement apparatus 200 shown in FIG. 12 is merely an implementation in embodiments of this application. In practice, the blood pressure measurement apparatus 200 may alternatively include more or fewer components than those shown in the figure, or a combination of a part of the components, or splits from a part of the components, or an arrangement of different components. This is not limited herein.

It should be understood that the steps in the foregoing method embodiments may be implemented by using a hardware integrated logical circuit in the processor or instructions in a form of software. The steps of the method disclosed with reference to embodiments of this application may be directly performed by a hardware processor, or may be performed through a combination of hardware in the processor and a software module.

This application further provides an electronic device. The electronic device may include a memory and a processor. The memory may be configured to store a computer program. The processor may be configured to invoke the computer program in the memory, to enable the electronic device to perform the method performed by the electronic device 100 in any one of the foregoing embodiments.

This application further provides a chip system. The chip system includes at least one processor, configured to implement functions in the method performed by the electronic device 100 in any one of the foregoing embodiments.

In a possible design, the chip system further includes a memory. The memory is configured to store program instructions and data. The memory is located inside the processor or outside the processor.

The chip system may include a chip, or may include a chip and another discrete component.

Optionally, there may be one or more processors in the chip system. The processor may be implemented by using hardware, or may be implemented by using software. When the processor is implemented by using the hardware, the processor may be a logic circuit, an integrated circuit, or the like. When the processor is implemented by using the software, the processor may be a general-purpose processor, and is implemented by reading software code stored in the memory.

Optionally, there may also be one or more memories in the chip system. The memory may be integrated with the processor, or may be disposed separately from the processor. This is not limited in embodiments of this application. For example, the memory may be a non-transitory processor, for example, a read-only memory ROM. The memory and the processor may be integrated into a same chip, or may be separately disposed on different chips. A type of the memory and a manner of disposing the memory and the processor are not specifically limited in embodiments of this application.

For example, the chip system may be a field programmable gate array (field programmable gate array, FPGA), an application-specific integrated circuit (application-specific integrated circuit, ASIC), a system on chip (system on chip, SoC), a central processing unit (central processing unit, CPU), a network processor (network processor, NP), a digital signal processor (digital signal processor, DSP), a micro controller unit (micro controller unit, MCU), a programmable logic device (programmable logic device, PLD), or another integrated chip.

This application further provides a computer program product. The computer program product includes a computer program (which may also be referred to as code or instructions). When the computer program is run, a computer is enabled to perform any method performed by the electronic device 100 in any one of the foregoing embodiments.

This application further provides a computer-readable storage medium. The computer-readable storage medium stores a computer program (which may also be referred to as code or instructions). When the computer program is run, a computer is enabled to perform any method performed by the electronic device 100 in any one of the foregoing embodiments.

It should be noted that the processor in embodiments of this application may be an integrated circuit chip, and has a signal processing capability. In an implementation process, the steps in the foregoing method embodiments may be implemented by using a hardware integrated logical circuit in the processor or instructions in a form of software. The processor may be a general-purpose processor, a digital signal processor (digital signal processor, DSP), an application-specific integrated circuit (application-specific integrated circuit, ASIC), a field programmable gate array (field programmable gate array, FPGA) or another programmable logic device, a discrete gate or a transistor logic device, or a discrete hardware component. It may implement or perform the methods, the steps, and logical block diagrams that are disclosed in embodiments of this application. The general-purpose processor may be a microprocessor, or the processor may be any conventional processor or the like. The steps of the method disclosed with reference to embodiments of this application may be directly performed by a hardware decoding processor, or may be performed through a combination of hardware in the decoding processor and a software module. A software module may be located in a mature storage medium in the art, for example, a random access memory, a flash memory, a read-only memory, a programmable read-only memory, an electrically erasable programmable memory, or a register. The storage medium is located in the memory, and a processor reads information in the memory and completes the steps in the foregoing methods in combination with hardware of the processor.

In addition, an embodiment of this application further provides an apparatus. The apparatus may be specifically a component or a module, and the apparatus may include one or more processors and memories that are connected to each other. The memory is configured to store a computer program. When the computer program is executed by one or more processors, the apparatus is enabled to perform the methods in the foregoing method embodiments.

The apparatus, the computer-readable storage medium, the computer program product, or the chip provided in embodiments of this application are all configured to perform the corresponding method provided above. Therefore, for beneficial effects that can be achieved, refer to beneficial effects in the corresponding method provided above, and details are not described herein again.

The implementations of this application may be randomly combined, to achieve different technical effects.

All or a part of the foregoing embodiments may be implemented by using software, hardware, firmware, or any composition thereof. When software is used to implement embodiments, all or a part of embodiments may be implemented in a form of computer program product. The computer program product includes one or more computer instructions. When the computer program instructions are loaded and executed on the computer, the procedure or functions according to this application are all or partially generated. The computer may be a general-purpose computer, a dedicated computer, a computer network, or other programmable apparatuses. The computer instructions may be stored in a computer-readable storage medium, or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line) or wireless (for example, infrared, radio, or microwave) manner. The computer-readable storage medium may be any usable medium accessible by the computer, or a data storage device, for example, a server or a data center, integrating one or more usable media. The usable medium may be a magnetic medium (for example, a floppy disk, a hard disk drive, or a magnetic tape), an optical medium (for example, a DVD), a semiconductor medium (for example, a solid-state drive (solid-state disk, SSD)), or the like.

A person of ordinary skill in the art may understand that all or some of the procedures of the methods in embodiments may be implemented by a computer program instructing related hardware. The program may be stored in the computer-readable storage medium. When the program is run, the processes of the methods in embodiments are performed. The foregoing storage medium includes any medium that can store program code, such as a ROM, a random access memory RAM, a magnetic disk, or an optical disc.

In conclusion, the foregoing descriptions are merely embodiments of the technical solutions of the present invention, but are not intended to limit the protection scope of the present invention. Any modification, equivalent replacement, improvement, or the like made in accordance with the disclosure of the present invention shall be included in the protection scope of the present invention.

## Claims

1. A blood pressure measurement method, wherein the method is applied to an electronic device, and the method comprises:
detecting a blood pressure measurement operation;
determining, based on historically collected first data and/or second data collected after the blood pressure measurement operation is detected, whether a user has an irregular heartbeat symptom; and
when the user has the irregular heartbeat symptom, determining a blood pressure value of the user based on blood pressure values of a plurality of measurements.

2. The method according to claim 1, wherein the second data is a photoplethysmography PPG signal or an oscillation wave signal.

3. The method according to claim 1 or 2, wherein whether the user has the irregular heartbeat symptom is determined based on the second data collected after the blood pressure measurement operation is detected, and
after detecting the blood pressure measurement operation, and before determining, based on the second data collected after the blood pressure measurement operation is detected, whether the user has the irregular heartbeat symptom, the method further comprises:
starting a 1^{st} blood pressure measurement, wherein the second data is data collected during the 1^{st} blood pressure measurement; and
after determining, based on the second data collected after the blood pressure measurement operation is detected, whether the user has the irregular heartbeat symptom, the method further comprises:
when it is determined, based on the second data, that the user has the irregular heartbeat symptom, starting N blood pressure measurements after the 1^{st} blood pressure measurement ends, wherein the blood pressure values of the plurality of measurements comprise blood pressure values respectively obtained through the 1^{st} blood pressure measurement and the N blood pressure measurements, and N is a positive integer greater than or equal to 1; or
when it is determined, based on the second data, that the user has the irregular heartbeat symptom, starting M blood pressure measurements after the 1^{st} blood pressure measurement ends, wherein the blood pressure values of the plurality of measurements comprise blood pressure values respectively obtained through the M blood pressure measurements, and M is a positive integer greater than or equal to 2.

4. The method according to claim 3, wherein when the 1^{st} blood pressure measurement is started, a blood pressure measurement mode is a first mode, and a blood pressure measurement mode of the N blood pressure measurements is a second mode; and
the electronic device is a wearable device configured with an airbag, and a pressure applied when the airbag is inflated in the first mode is lower than a pressure applied when the airbag is inflated in the second mode; and/or
a blood pressure measurement model used in the first mode is different from a blood pressure measurement model used in the second mode, and the blood pressure measurement model is used to determine a blood pressure value of the measurement.

5. The method according to claim 4, wherein after starting the 1^{st} blood pressure measurement, the method further comprises:
during the 1^{st} blood pressure measurement, determining that the user has the irregular heartbeat symptom, and switching the blood pressure measurement mode of the 1^{st} blood pressure measurement from the first mode to the second mode.

6. The method according to claim 1, wherein the first data comprises a PPG signal.

7. The method according to claim 1 or 6, wherein whether the user has the irregular heartbeat symptom is determined based on the historical first data of the user, and
before determining the blood pressure value of the user based on the blood pressure values of the plurality of measurements for the user, the method further comprises:
starting a plurality of blood pressure measurements, wherein the blood pressure values of the plurality of measurements are blood pressure values obtained through the plurality of blood pressure measurements.

8. The method according to claim 7, wherein the method further comprises:
when it is determined, based on the first data, that the user does not have the irregular heartbeat symptom, starting one blood pressure measurement, and determining a blood pressure value obtained through the one blood pressure measurement as the blood pressure value of the user.

9. The method according to claim 8, wherein after starting the one blood pressure measurement, and before determining the blood pressure value obtained through the one blood pressure measurement as the blood pressure value of the user, the method further comprises:
determining, based on the second data collected during the one blood pressure measurement, that the user does not have the irregular heartbeat symptom.

10. The method according to claim 7, wherein the method further comprises:
when it is determined, based on the first data, that the user does not have the irregular heartbeat symptom, starting one blood pressure measurement; and
when it is determined, based on the second data collected during the one blood pressure measurement, that the user has the irregular heartbeat symptom, starting N blood pressure measurements after the one blood pressure measurement ends, wherein the blood pressure values of the plurality of measurements comprise blood pressure values respectively obtained through the one blood pressure measurement and the N blood pressure measurements, and N is a positive integer greater than or equal to 1; or
when it is determined, based on the second data, that the user has the irregular heartbeat symptom, starting M blood pressure measurements after the 1^{st} blood pressure measurement ends, wherein the blood pressure values of the plurality of measurements comprise blood pressure values respectively obtained through the M blood pressure measurements, and M is a positive integer greater than or equal to 2.

11. The method according to any one of claims 8 to 10, wherein a blood pressure measurement mode of the one blood pressure measurement is a first mode, and a blood pressure measurement mode of the plurality of blood pressure measurements is a second mode; and
the electronic device is a wearable device configured with an airbag, and a pressure applied when the airbag is inflated in the first mode is lower than a pressure applied when the airbag is inflated in the second mode; and/or
a blood pressure measurement model used in the first mode is different from a blood pressure measurement model used in the second mode, and the blood pressure measurement model is used to determine a blood pressure value of the measurement.

12. The method according to claim 4 or 11, wherein a deflation speed of the airbag in the first mode is greater than a deflation speed of the airbag in the second mode.

13. The method according to claim 4, 11, or 12, wherein the blood pressure values of the plurality of measurements are determined based on an oscillation wave signal collected by the electronic device in an airbag inflation process.

14. The method according to any one of claims 1 to 13, wherein
in the blood pressure values of the plurality of measurements, a difference between blood pressure values of any two measurements is less than a first threshold, and/or a heart rate value of the user during each measurement is less than a second threshold, and/or a heart rate difference of the user during any two measurements is less than a third threshold.

15. The method according to any one of claims 1 to 14, wherein when the user has the irregular heartbeat symptom, after determining the blood pressure value of the user based on the blood pressure values of the plurality of measurements, the method further comprises:
displaying the blood pressure value of the user and a measurement identifier, wherein the measurement identifier indicates that the blood pressure value of the user is determined when the user has the irregular heartbeat symptom.

16. The method according to any one of claims 1 to 15, wherein determining the blood pressure value of the user based on the blood pressure values of the plurality of measurements specifically comprises:
determining an average of the blood pressure values of the plurality of measurements as the blood pressure value of the user.

17. An electronic device, comprising a memory, one or more processors, and one or more programs, wherein when the one or more processors execute the one or more programs, the electronic device is enabled to implement the method according to any one of claims 1 to 16.

18. A computer-readable storage medium, comprising instructions, wherein when the instructions are run on an electronic device, the electronic device is enabled to perform the method according to any one of claims 1 to 16.

19. A computer program product, wherein when the computer program product runs on a computer, the computer is enabled to perform the method according to any one of claims 1 to 16.
